# EUROPEAN PATENT SPECIFICATION

(11) **EP 2 568 052 B1**
(45) Date of publication and mention of the grant of the patent: **07.09.2016**
(21) Application number: 11777513.0
(22) Date of filing: 20.04.2011
(51) Int. Cl.: C12Q 1/68, C12Q 1/70, G01N 30/72

(54) **METHOD FOR PRODUCING AN AMPLICON COMPRISING A GENE REGION HAVING VARIOUS VARIATIONS AND BEING USEFUL IN MASS SPECTROMETRY**
VERFAHREN ZUR HERSTELLUNG EINES AMPLIKONS DAS EINE GENREGION MIT VERSCHIEDENEN VARIATIONEN ENTHÄLLT UND DURCH MASSENSPEKTROMETRIE ANALYSIERT WERDEN KANN
PROCÉDÉ PERMETTANT DE PRODUIRE UN AMPLICON COMPRENANT UNE RÉGION GÉNIQUE PRÉSENTANT DIVERSES MODIFICATIONS ET SOYANT ANALYSABLE PAR SPECTROMETRIE DE MASSE

(30) Priority: 07.05.2010 KR 20100043166
(43) Date of publication of application: 13.03.2013
(73) Proprietor: Genematrix Inc., Gyeonggi-do 463-400 (KR)
(72) Inventor: YOO, Wangdon, Seoul 140-752 (KR); HWANG, Sun Young, Seoul 135-240 (KR); SHIN, Soo-Kyung, Suwon-si Kyeonggi-do 441-703 (KR); SHIN, Ji Young, Suwon-si Kyeonggi-do 443-706 (KR); LEE, Joo Hyoung, Seoul 135-850 (KR); PARK, Sun Min, Seongnam-si Kyeonggi-do 463-792 (KR); HONG, Sun Pyo, Seoul 137-060 (KR); KIM, Sukjoon, Seoul 157-280 (KR); KIM, Soo-Ok, Seoul 138-162 (KR)
(74) Representative: Isarpatent
(86) International application number: PCT/KR2011/002805
(87) International publication number: WO 2011/139032

(56) References cited:
- EP-A1- 2 055 791
- WO-A1-2004/035832
- WO-A2-02/20845
- WO-A2-2005/038039
- WO-A2-2007/147063
- SUN PYO HONG ET AL: "High-resolution human papillomavirus genotyping by MALDI-TOF mass spectrometry", NATURE PROTOCOLS, NATURE PUBLISHING GROUP, GB, vol. 3, no. 9, 28 August 2008 (2008-08-28) , pages 1476-1484, XP008162805, ISSN: 1750-2799, DOI: 10.1038/NPROT.2008.136 [retrieved on 2008-08-28]
- HONG, S. P. ET AL.: 'High-resolution human papillomavirus genotyping by MALDI-TOF mass spectrometry' NAT. PROTOC. vol. 3, no. 9, 2008, pages 1476 - 1484, XP008162805
- SOHN, Y. H. ET AL.: 'Performance evaluation of the Abbott RealTime HCV Genotype II for hepatitis C virus genotyping' CLIN. CHEM. LAB. MED. vol. 48, no. 4, April 2010, pages 469 - 474, XP008162748
- KWON, H. C. ET AL.: 'Emergence of adefovir-resistant mutants after reversion to YMDD wild-type in lamivudine-resistant patients receiving adefovir monotherapy' J. GASTROENTEROL. HEPATOL. vol. 24, no. 1, January 2009, pages 49 - 54, XP008162718
- WOO, H. Y. ET AL.: 'Evaluation of dual priming oligonucleotide-based multiplex PCR for detection of HBV YMDD mutants' ARCH. VIROL. vol. 153, no. 11, 05 October 2008, pages 2019 - 2025, XP019660409
- OH, H. B. ET AL.: 'Identification of hepatitis C virus genotype 6 in Korean patients by analysis of 5' untranslated region using a matrix assisted laser desorption/ionization time of flight-based assay, restriction fragment mass polymorphism' J. MED. VIROL. vol. 80, no. 10, October 2008, pages 1712 - 1719, XP008162722
- LEE, E. H. ET AL.: 'Human papilloma virus genotyping assay using restriction fragment mass polymorphism analysis, and its comparison with sequencing and hybrid capture assays' KOREAN J. LAB. MED vol. 27, no. 1, February 2007, pages 62 - 68, XP008162745
- HWANG, S. H. ET AL.: 'Effective screening of informative single nucleotide polymorphisms using the novel method of restriction fragment mass polymorphism' J. INT. MED. RES. vol. 35, no. 6, November 2007, pages 827 - 835, XP008162739

## Description

### TECHNICAL FIELD

The present disclosure relates to a primer composition for amplifying a gene region having various variations in a target gene, a method for amplifying a target gene using the same, a PCR amplification kit comprising the same, and a method for genotyping a target gene, more particularly to a primer composition capable of amplifying a gene region having various variations simply and efficiently in a target gene to accomplish the accurate amplification of the gene region without missing and to analyze a number of genotypes precisely with satisfying both specificity and sensitivity, a method for amplifying a target gene using the same, a PCR amplification kit comprising the same, and a method for genotyping a target gene using the same.

In addition, the present disclosure relates to a primer composition capable of generating amplification products whose sequences are identical with each other except for "genotype-analyzable sequence" of the sequences subjected to be analyzed when amplifying a gene region having various variations in a target gene, to reduce the number of peaks shown in genotyping based on the restriction fragment mass polymorphism (RFMP) assay and to facilitate the genotyping, a method for amplifying a target gene using the same, a PCR amplification kit comprising the same, and a method for genotyping a target gene using the same.

### BACKGROUND ART

The genotyping of live organism is widely used in relation with the measurement of the degree of risk, the preliminary medical examination or diagnosis of diseases to provide an appropriate treating method therefor. For example, it can lead to prevent a disease in advance by analyzing variations of a particular gene for a subject and predicting the degree of risk of the disease. In addition, it can be performed to analyze whether any infectious agent causing an infection in an organism such as a virus is resistant to a particular treatment medicine through the analysis of genetic variations of the virus, and to predict the response of the infectious agent to the particular treatment medicine, for example, the resistance response to the medicine and thus to develop a patient-tailored treatment method.

After completing the genetic map of human genome, it is allowed to widely conduct the measurement of the degree of risk of diseases, the preliminary medical examination or diagnosis of diseases and the prediction of response to a medicine, and to make the interest of genotyping be increased in the medical field. However, it is noted that a number of base sequence informations should be analyzed and the anaylsis should be performed accurately and efficiently with satisfying both specificity and sensitivity in order to clearly reveal the relationship between genetic mutations and diseases in living organisms including human being.

Although there are several methods to analyze variations of the nucleotide sequences or bases, basically a large amount of genetic samples should be obtained before using the methods. Currently, the most common method for obtaining genetic samples is to use the polymerase chain reaction (PCR) method using DNA polymerase (hereinafter, referred to as "PCR"). According to the aforesaid method, a desired region of a gene can be amplified by designing primers that can bind to a template. The primers are designed by selecting a region subjected to be amplified from a gene and determining nucleotide sequences capable of complementarily binding to 3' end sequences of the selected region.

However, it is difficult to design primers for a gene region having various variations in a target gene selected for genotyping. It requires careful handling and sufficient time to synthesize PCR primers. Besides, there is a problem that false positive products are generated by non-specific hybridization of primers. Especially, if there are dozens or hundreds of genotypes in a target gene, it is more difficult to precisely amplify all of gene regions each of which having various variations in a target gene and to analyze the genotypes thereof with satisfying both specificity and sensitivity. For example, it is known that human papillomavirus (hereinafter, referrred to as "HPV") has more than 80 strains and about 120 subtypes.

If there are at least dozens of genotypes in a target gene as mentioned above, it is difficult to design a plurality of primers precisely for a gene region having various variations in a target gene. In addition, it would cause false negative problem to determine the genotype of interest as negative due to failing to amplify a gene region having a specific variation, or positive false problem by the aforesaid non-specific hybridization of primers. The positive false problem becomes more serious with increasing of the number of kinds of used primers. In terms of a trade-off between sensitivity and specificity, it is difficult to design and provide a plurality of primers with satisfying both sensitivity and specificity so as to precisely amplify a gene region having various variations in a target gene, provided that there are at least dozens of genotypes in a target gene.

It is known that a method for designing primers to contain at least one mixed base or to mix a plurality of designed primers has been generally used when amplifying a gene region having various variations.

First of all, if it is assumed that there are A-type and B-type genes subjected to be analyzed as follows, the primers containing at least one mixed base are prepared in such a manner that the third and fifteenth bases are designed using mixed bases corresponding to adenine (A) or cytosine (C), and guanine (G) or adenine (A), respectively:
A-type gene: 5'-ggA ttc ggg ccc gaG tct-3'; and
B-type gene: 5'-ggC ttc ggg ccc gaA tct-3'.

Accordingly, the primer having the following sequence can be designed for the A-type and B-type genes: 5'-ggM ttc ggg ccc gaR tct-3' (R = A/G, M = A/C).

However, if a lot of mixed bases are used, Tm value of the primer becomes higher and bases that differ from the nucleotide sequence of the template can be synthesized to produce serveral amplified products of which the nucleotide sequences differ from one template due to using the mixed bases. Therefore, the prior art using the mixed bases can not solve the aforesaid positive false problem of PCR products.

Alternatively, it is provided that a primer A specific to the A-type gene and a primer B specific to the B-type gene are designed, respectively and mixed together to produce PCR products by binding the primers A and B to the A-type and B-type genes, respectively. This method could work if a gene region has a few variations in a target gene. However, it would cause the false positive problem when amplifying a gene region having various variations in a target gene if the gene region has more than dozens of variations in the target gene. For example, it is usually expected that the primers A and B bind to the A-type and B-type genes, respectively to produce their own PCR products in the above case. But, there is the possibility that the products of the primers A and B can be amplified together from one template. In the concentration of the initial trace amount of a template, a specific primer can only bind to the corresponding template, however, a non-specific primer can also bind to the products amplified from the template after amplifying the products upto a certain concentration. This has resulted in mixing several primers for one template and amplifying several products of which the nucleotide sequences differ from each other.

Therefore, if primers adopting mixed bases are used or several specific primers are mixed at the same concentration when amplifying a gene region having various variations by PCR which is previously performed to analyze the nucleotide sequences of the template, it is impossible to accurately analyze the nucleotide sequences due to replicating the template by non-specific primers. In other words, it is difficult to provide primers with satisfying both sensitivity and specificity when amplifying a gene region having various variations if the primers are simply designed based on a one-to-one correspondence relationship for each genotype. On the other hand, if the kinds of used primers are minimized, the false positive problem could be solved to some extent, but the problem of sensitivity decrease would inevitably occur with being not capable of amplifying a specific variation.

Meanwhile, if several PCR products, of which the nucleotide sequences differ from each other, are amplified from one template, it could lead to the false result in analyzing the nucleotide sequences of the template. For example, in PCR-SSCP, it is performed to amplify nucleotide sequences including bases subjected to be analyzed, to separate them into each chain and to run them in polyacryl amide gel electrophoresis (Orita, M. et.al, Genomics, 1989, 5:8874-8879). In the PCR-SSCP, since one nucleotide difference results in the difference of the secondary structure of DNA chain, it is possible to identify whether there is a base variation depending on electrophoretic mobility or speed difference resulted from the difference of the secondary structure. In accordance with the PCR-SSCP method, it is possible to accurately identify the base variation when the base subjected to be analyzed is only replicated from the template, with the same base sequence in the remain. Accordingly, if several products, of which the nucleotide sequences differ from each other, are amplified, it can lead to erroneous results.

In addition, a new genotyping method of so-called "Restriction Fragment Mass Polymorphism assay (RFMP assay)" is disclosed in Korean Patent Publication Nos. 10-0477766 and 10-0642829. In the RFMP assay, it is performed to amplify nucleotide sequences including bases subjected to be analyzed, to excise them by restriction enzymes to produce fragments, and to analyze the genotype by measuring the molecular weights of the fragments. Howerver, if several products, of which the nucleotide sequences differ from each other, are amplified, the problem would occur that several molecular weights are dfferently measured from one template. In addition, since the RFMP is a method to analyze the genotype by measuring the molecular weights of the fragments from the amplified products of the gene region subjected to be analyzed, if there are many molecular weight peaks in the mass spectrometry graph, it makes the RFMP assay be complex and difficult.

WO-A-2007/147063 discloses a method for amplifying different alleles for mass spectrometry wherein primers are used in different concentrations in order to ensure that the amplicons of each allele are present in equal amounts.

Accordingly, if a plurality of primers are prepared based on a one-to-one correspondence relationship for each genotype and then mixed to amplify a gene region having various variations in a target gene and to analyze the genotype of the gene region by the RFMP assay, there is a high possibility that its analysis becomes more difficult and leads to the erroneous results since the mass spectrometry peaks are increased.

In order to solve the aforesaid problems, it needs in the art to provide a method with satisfying both sensitivity and specificity in terms of a trade-off and thus to precisely amplify a gene region having various variations in a target gene.

### SUMMARY OF INVENTION

The object of the present invention is to solve the aforesaid problems and to provide a method, as defined in claim 1, capable of amplifying a gene region having various variations simply and efficiently in a target gene to accomplish the accurate amplification of the gene region without missing and to analyze a number of genotypes precisely with satisfying both specificity and sensitivity More particularly, the object of the present invention is to provide a method, as defined in claim 1, comprising primers obtained by designing and mixing each specific primer when there are various variations in bases near sequences subjected to be analyzed and capable of amplifying all the rest except for "genotype-analyzable sequences" of the sequences to have the same sequences, to enable the sequences having various genetic variations to be amplified through PCR and to make the sequences and variations thereof be precisely analyzed.

Further, the method of the present invention provides amplification products whose sequences are identical with each other except for "genotype-analyzable sequences" of the sequences subjected to be analyzed when amplifying a gene region having various variations in a target gene, to reduce the number of peaks shown in genotyping based on the restriction fragment mass polymorphism (RFMP) assay and to facilitate the genotyping.

### DETAILED DESCRIPTION OF INVENTION

The inventors of the present invention had continued to study the improved RFMP assay in relation with the aforesaid problems. As a result, the inventors of the present invention found that it is difficult to provide primers satisfying both sensitivity and specificity if the primers are simply designed based on a one-to-one correspondence relationship for each genotype when amplifying a gene region having various variations in a target gene, and it is possible to accurately analyze nucleotide sequences of the gene region by minimizing used primers and amplifying all the rest except for "genotype-analyzable sequences" to have the same sequences.

Meanwhile, the primer composition capable of amplifying a gene region having various variations is not limited to the RFMP assay as described in one embodiment, but it should be explained as a platform technology that is applicable to a variety of techniques for analyzing genotypes using PCR products, for example, a genotyping technology such as microarrayed chip, etc.

First, the terms used in the specification of the present invention will be described as follows.

The restriction fragment mass polymorphism assay (hereafter, referred to as "RFMP assay") used in the specification means a genotyping method to amplify a gene subjected to be analyzed by PCR, to excise the PCR products using restriction enzymes recognizing the restriction sites having variations, to produce restricted fragments that differ in their molecular weights depending on the presence of base variations and to analyze genotypes by measuring the molecular weights of the restricted fragments (see Korean Patent Publication Nos. 10-0477766 and 10-0642829).

The restriction enzyme recognition sequence used in the specification of the present invention means a sequence recognized at the same time or closely by different restriction enzymes when performing the aforesaid RFMP assay and that may not match with a sequence excised by the restriction enzymes. In an embodiment of the present invention as described in detail, the restriction enzymes of FokI and BtsCI used as a example recognize GGATG sequence but excise the position next to 9th/13th and 2nd/ 0th bases from the 3 'end of the recognized sequence, respectively. In addition, all the restriction enzymes can be used as two restriction enzymes recognizing the restriction enzyme recognition sequence. For example, two restriction enzymes that can be used for the purposes of the present invention may include restriction enzymes having the same optimal temperature or different optimal temperatures to each other, preferably, having different optimal temperatures among them. For example, a restriction enzyme having a relatively low optimal temperature may include FokI, BbvI, BsgI, BcgI, BpmI, BseRI Mme1I, AvaII or BaeI, etc. For example, a restriction enzyme having a relatively high optimal temperature may include BtsCI , BstF5I, TaqI, BsaBI, BtrI, BstAPI, FauI, BclI, PciI, or ApoI etc. (see Korean Patent Publication Nos. 10-0477766 and 10-0642829).

In addition, the term of "trigger primer" (TP; also referred to as a first primer group) used in the specification means a primer specific to a corresponding template including a genotype-analyzable sequence and that is mixed in a PCR reaction mixture at a low concentration so as to be completely exhausted in the initial PCR reaction.

Further, the term of "amplification primer" (AP; also referred to as a second primer group) used in the specification means a primer which is prepared by aligning sequences in a target gene to determine a genotype-analyzable sequence and by selecting a primer-binding portion. The amplification primer is designed in such a manner that it can bind specifically to a specific template or bind complementarily to a variety of templates with allowing mismatch of up to three successive bases or preferably 1 to 2 bases in the vicinity of the 3' ends of the templates. The amplification primer is mixed in a PCR reaction mixture at a high concentration to produce the final PCR products using the initial PCR products produced by the trigger primers as templates.

Usually, a primer composition is provided by preparing several trigger primers each of which is specific to the corresponding sequence in the vicinity of the 3' ends of the templates including genotype-analyzable sequences in a number of target genes and by selecting one primer having the greatest number of shared bases among the trigger primers to determine at least one amplification primer.

Furthermore, the term of "genotype-analyzable sequence" used in the specification means a sequence (a nucleotide sequence or an amino acid sequence) capable of specifically distinguishing a genotype of a target gene of interest from the other genotype, and that is present in a region having various variations subjected to be analyzed in the target gene.

The present invention provides a method, as defined in the claims.

The concentration of the first primer group provided to the first amplification reactant may be adjusted to a certain concenration in such a manner that the primer of the first primer group can bind to the template in the initial amplification reaction of the template (for example, 10 to 15 PCR amplification cylces) and be exhausted after amplifying the amplification products of the template upto a certain concentration.

For example, the concentration of the primer of the first primer group may be preferably 1/2 to 1/20, more preferably 1/8 to 1/16 of the concentration of the primer of the second primer group. However, the present invention is not limited hereto, and the concentration of the primer may vary depending on the reaction conditions.

In one embodiment the total concentration of the primers of the first primer group and the second primer group is preferably within 2µM.

In one embodiment more than four bases of the primer of the first primer group should be complementary to the nucleotide sequence of the 3' end portion of the template. In addition, if the primer is mismatched with the template over three successive bases in the nucleotide sequence of the other portion, it is difficult for the primer to bind with the template.

Each primer of the first primer group is designed in such a manner that its nucleotide sequence having a base variation can bind complementarily to the template and the other sequence part is identical with the primer of the second primer group as a common sequence. Also, the primer of the first primer group has the same sequence length as the selected primer of the second primer group.

The final amplification products are amplified to have the same nucleotide sequences to each other except for the genotype-analyzable sequence.

In one embodiment of the method, the primer of the first primer group is at least one primer selected from the group consisting of SEQ ID NO: 6 and SEQ ID NOs: 8-10, and the primer of the second primer group is at least one primer selected from the group consisting of SEQ ID NO: 5 and SEQ ID NO: 7 when the target gene is HPV gene.

In one embodiment of the disclosure the primer of the first primer group is at least one primer selected from the group consisting of SEQ ID NO: 25 and SEQ ID NO: 26, and the primer of the second primer group is at least one primer selected from the group consisting of SEQ ID NO: 23 and SEQ ID NO: 24 when the target gene is HLA-DQB1 gene.

In one embodiment of the disclosure, the primer of the first primer group is at least one primer selected from the group consisting of SEQ ID NOs: 42-48, and the primer of the second primer group is at least one primer selected from the group consisting of SEQ ID NO: 40 and SEQ ID NO: 41 when the target gene is HBV gene.

In addition, it is not necessary to provide the first primer group as a pair of a forward primer and a reverse primer. For example, if a reverse primer is only provided for the first primer group, the reverse primer would pair the forward primer of the second primer group to perform the amplification reaction. The second primer group is provided to the amplification reactant as the combination of a forward primer and a reverse primer.

The present disclosure provides a PCR amplification kit including a primer composition for amplifying a gene region having various variations in a target gene comprising: at least one polynucleotide template; a first primer group comprising at least one primer specific to at least one template including a genotype-analyzable sequence of a genetically variant base sequence subjected to be analyzed in the target gene; a second primer group comprising at least one primer designed by selecting one primer having the greatest number of shared bases from the primers of the first primer group and by using the selected primer as a reference, the primer of the second primer group binding specifically to the template or binding complementarily to the template with allowing mismatch of up to three successive bases, or 1 to 2 bases in the vicinity of the 3' end of the template; a DNA polymerase; dNTPs; and a buffer solution.

In one aspect of the disclosure of the PCR amplification kit, the first primer group is provided to a first amplification reactant at a low concentration to be completely exhausted in an initial amplification reaction of the template, and the second primer group is provided to a second amplification reactant at a high concentration to produce final amplification products using the initial amplification products amplified by the first primer group as templates.

In one aspect of the disclosure of the PCR amplification kit, the concentration of the first primer group provided to the first amplification reactant may be preferably adjusted to a certain concenration in such a manner that the primer of the first primer group can bind to the template in the initial amplification reaction of the template (for example, 10 to 15 PCR amplification cylces) and be exhausted after amplifying the amplification products of the template upto a certain concentration. For example, the concentration of the primer of the first primer group may be preferably 1/2 to 1/20, more preferably 1/8 to 1/16 of the concentration of the primer of the second primer group. However, the present invention is not limited hereto, and the concentration of the primer may vary depending on the reaction conditions.

The present invention provide a method for amplifying a target gene as defined in claim 1.

In the method for amplifying the target gene, the first primer group is added to the amplification reactant at a low concentration, and the second primer group is added to the amplification reactant at a high concentration as compared with the first primer group in the (c) step.

The concentration of the primer of the first primer group may be preferably 1/2 to 1/20, more preferably 1/8 to 1/16 of the concentration of the primer of the second primer group in the (c) step. However, the present invention is not limited hereto, and the concentration of the primer may vary depending on the reaction conditions.

In one embodiment of the method for amplifying the target gene, the total concentration of the primers of the first primer group and the second primer group is preferably within 2µM in the (c) step.

In one embodiment of the method for amplifying the target gene, the primer of the first primer group is at least one primer selected from the group consisting of SEQ ID NO: 6 and SEQ ID NOs: 8-10, and the primer of the second primer group is at least one primer selected from the group consisting of SEQ ID NO: 5 and SEQ ID NO: 7 when the target gene is HPV gene.

In one aspect of the disclosure of the method for amplifying the target gene, the primer of the first primer group is at least one primer selected from the group consisting of SEQ ID NO: 25 and SEQ ID NO: 26, and the primer of the second primer group is at least one primer selected from the group consisting of SEQ ID NO: 23 and SEQ ID NO: 24 when the target gene is HLA-DQB1 gene.

In one aspect of the disclosure of the method for amplifying the target gene, the primer of the first primer group is at least one primer selected from the group consisting of SEQ ID NOs: 42-48, and the primer of the second primer group is at least one primer selected from the group consisting of SEQ ID NO: 40 and SEQ ID NO: 41 when the target gene is HBV gene.

In the method for amplifying the target gene, the second primer group is added to the amplification reactant as the combination of a forward primer and a reverse primer.

The present invention provide a method for genotyping a target gene using a primer composition for amplifying a gene region having various variations in a target gene comprising:
(a) amplifying the target gene according to the method for amplifying the target gene as mentioned above;
(b) excising a polynucleotide amplified in the (a) step by restriction enzymes, the polynucleotide including a genotype-analyzable sequence of a genetically variant base sequence subjected to be analyzed in the target gene;
(c) generating at least two kinds of single strand polynucleotide fragments having the variant base by the excision of the restriction enzymes, the number of bases of the fragments being within a limited range; and
(d) measuring the molecular weights of the fragments.

In one embodiment of the method for genotyping the target gene, the number of bases of the fragments ranges between 2 and 32.

In one embodiment of the method for genotyping the target gene, the primer of the first primer group is at least one primer selected from the group consisting of SEQ ID NO: 6 and SEQ ID NOs: 8-10, and the primer of the second primer group is at least one primer selected from the group consisting of SEQ ID NO: 5 and SEQ ID NO: 7 when the target gene amplified in the (a) step is HPV gene.

In one aspect of the disclosure of the method for genotyping the target gene, the primer of the first primer group is at least one primer selected from the group consisting of SEQ ID NO: 25 and SEQ ID NO: 26, and the primer of the second primer group is at least one primer selected from the group consisting of SEQ ID NO: 23 and SEQ ID NO: 24 when the target gene amplified in the (a) step is HLA-DQB1 gene.

In one aspect of the disclosure of the method for genotyping the target gene, the primer of the first primer group is at least one primer selected from the group consisting of SEQ ID NOs: 42-48, and the primer of the second primer group is at least one primer selected from the group consisting of SEQ ID NO: 40 and SEQ ID NO: 41 when the target gene amplified in the (a) step is HBV gene.

In the method for genotyping the target gene, the second primer group for the target gene amplified in the (a) step is provided as the combination of a forward primer and a reverse primer.

### ADVANTAGEOUS EFFECTS

The present invention can amplify a gene region having various variations simply and efficiently in a target gene to accomplish the accurate amplification of the gene region and to analyze a number of genotypes precisely whilst satisfying both specificity and sensitivity.

More particularly, the present invention adopting trigger primers and amplification primers in PCR reaction can generate amplification products whose sequences are identical with each other except for "genotype-analyzable sequences" of the sequences subjected to be analyzed when amplifying a gene region having various variations in a target gene, to enable the sequences having various genetic variations to be amplified through PCR and to make the sequences and variations thereof be precisely analyzed.

Further, according to the present invention, it is possible to generate amplification products whose sequences are identical with each other except for "genotype-analyzable sequences", to reduce the number of peaks shown in genotyping based on the restriction fragment mass polymorphism (RFMP) assay and to facilitate the genotyping.

Accordingly, the present invention can be used in a variety of fields, for example, biotechnology field, medical field and pharmaceutical field required for analyzing base variations.

### BRIEF DESCRIPTION OF DRAWINGS

The above and other objects, features and other advantages of the present invention will be more clearly understood to those skilled in this arts from the following detailed description taken in conjunction with the accompanying drawings.
FIG. 1 represents a schematic diagram that explains a method for amplifying various variant genes simply and efficiently without missing and thus analyzing a number of genotypes by performing PCR reaction, using trigger primers and amplification primers.
FIG. 2 represents a graph of MALDI-TOF mass spectrometry for HPV 59 genotype obtained by performing PCR amplification using trigger primers and amplification primers and then conducting the RFMP assay.
FIG. 3 represents a graph of MALDI-TOF mass spectrometry for HPV 16 genotype obtained by performing PCR amplification using trigger primers and amplification primers and then conducting the RFMP assay.
FIG. 4 represents a graph of MALDI-TOF mass spectrometry for HPV 51 genotype obtained by performing PCR amplification using trigger primers and amplification primers and then conducting the RFMP assay.
FIG. 5 represents a graph of MALDI-TOF mass spectrometry for HPV 68 genotype obtained by performing PCR amplification using trigger primers and amplification primers and then conducting the RFMP assay.
FIG. 6 represents a graph of MALDI-TOF mass spectrometry for human hetero-genotype of HLA-DQB1 (0201/0501) obtained by performing PCR amplification using trigger primers and amplification primers and then conducting the RFMP assay.
FIG. 7 represents a graph of MALDI-TOF mass spectrometry for human hetero-genotype of HLA-DQB1 (0301/0401) obtained by performing PCR amplification using trigger primers and amplification primers and then conducting the RFMP assay.
FIG. 8 represents a graph of MALDI-TOF mass spectrometry, in the event that HBV polymerase inducing adefovir-resistance by its base variation shows wild-type HBV genotype where its 236th condon is AAC for coding the amino acid of asparagine (Asn), obtained by performing PCR amplification using trigger primers and amplification primers and then conducting the RFMP assay.
FIG. 9 represents a graph of MALDI-TOF mass spectrometry, in the event that HBV polymerase inducing adefovir-resistance by its base variation shows mutant-type HBV genotype where its 236th condon is ACT for coding the amino acid of threonine (Thr), obtained by performing PCR amplification using trigger primers and amplification primers and then conducting the RFMP assay.

### EXAMPLES

Practical and presently preferred embodiments of the present invention are illustrated more clearly as shown in the following examples.

### Example 1. Genotyping HPV (Human Papillomavirus)

The genotyping of human papillomavirus (hereinafter, referred to as "HPV") known to cause human cervical cancer was performed using the restriction fragment mass polymorphism assay (hereinafter, referred to as "RFMP assary") as described above. In this example, the assay was performed for HPV DNAs.

The HPV is a DNA virus that belongs to a family of papova viruses, and forms icosahedron consisting of 72 outer units (capsomers) and comprises double-stranded circular DNA consisting of 7,900 nucleotides. The HPV is divided into different subtypes of 120 strains, depending on their similarity of the nucleotide sequences.

30 strains of these 120 subtypes are known to infect lower genital tract. Depending on the degree of risk that causes cervical intraepithelial neoplasia and cervical cancer, they are divided into high risk type of HPV-16, HPV-18, HPV-26, HPV-30, HPV-31, HPV-33, HPV-35, HPV-39, HPV-45, HPV-51, HPV-52, HPV-53, HPV-56, HPV-57, HPV-58, HPV-59, HPV-67, HPV-68, HPV-73, HPV-74; and low risk type of HPV-2a, HPV-3, HPV-6, HPV-10, HPV-11, HPV-32, HPV-34, HPV-40, HPV-42, HPV-43, HPV-44. HPV-54, HPV-55, HPV-61, HPV-69, HPV-70 (Munoz N et al., N Engl J Med, 2003, 348 (6): 518-27).

For genotyping and testing of HPV, it is difficult to amplify dozens of HPV DNA in order to analyze their various mutations and especially to meet both specificity and sensitivity in testing the high-risk type.

Meanwhile, the genome structure of HPV is largely divided into early transcription region E (early gene region) and late transcription region L (late gene region), and LCR (long control region) that is a region not expressed.

The genome structure of HPV dominantly affects the pattern of onset and degree of risk of diseases, and the prognosis of diseases. Especially, the E6 and E7 genes of the early transcription region are known to play the most important role in carcinogenesis by being inserted into the genome of infected cells and expressed in staying therein.

For example, the E6 and E7 genes of HPV belonging to the high-risk type bind to proteins expressed from p53 tumor suppressor gene and rb (retinoblastoma) gene, respectively to inactivate them.

As a result, the regulation of cell cycle and cell death (apoptosis) mechanism are inhibited to transform normal cells into cancer cells in the cervix.

On the other hand, HPV belonging to the low-risk type such as HPV-6 and HPV-11 is known to have decreased ability to inactivate proteins expressed from tumor suppressor genes and it is difficult for HPV belonging to the low-risk type to cause cervical cancer (Barbosa M.S. et al., In vitro biological activities of the E6 and E7 genes vary among HPVs of different oncogenic potential. J. Virol., 65: 292-298, 1991).

Therefore, if a genetic variation is dectected in a targe gene such as the E6 and E7 genes to analyze the genotype of HPV, it is possible to examine high-risk HPV infection and thus to monitor the causes of cervical cancer in advance.

The genotyping method of HPV will be explained in accordance with the RFMP assay, which adopts the aforesaid trigger primers and amplification primers in the following examples.

### 1. PCR amplification

A pair of amplification primers (AP) were prepared by aligning sequences of about 80 HPV strains to determine "genotype-analyzable sequences" and by selecting a primer-binding portion.

The amplification primers were designed in such a manner that they can bind complementarily to a variety of HPV DNA templates with allowing mismatch of up to three successive bases or preferably 1 to 2 bases in the vicinity of the 3' ends of the templates.

For example, the amplification primers were determined by preparing several trigger primers (TP) each of which is specific to the corresponding sequence in the vicinity of the 3' ends of a variety of HPV DNA templates and by selecting one primer having the greatest number of shared bases among the trigger primers. The PCR was carried out using a mixture including a high concentration of the amplification primers (AP) and a low concentration of trigger primers (TP) as designed above.

The template DNA sequences (5' → 3') amplified by the primers in the high-risk type HPV genes are represented as follows:
**HPV 59**
**HPV 16**
**HPV 51**
**HPV 68**

The underlined sequences of the HPV DNA template sequences are binding portions of the primer Nos. 1-6 as below. The underlined sequence in front of each HPV DNA template is a binding portion of a forward primer. The underlined sequence in the rear of each HPV DNA template is a binding portion of a reverse primer. The sequence represented in lowercase is the genotype-analyzable sequence.

### * Forward primers

Primer 1. 5'-GCACAGGG**CCAC**AAggatgAATGG-3' (24mer) (SEQ ID NO: 5)
Primer 2. 5'-GCACAGGG**TTTA**AAggatgAATGG-3' (24mer) (SEQ ID NO: 6)

### * Reverse primers

Primer 3. 5'-GTAGTATCAACAACggatgTAA**C**AAA-3' (26mer) (SEQ ID NO: 7)
Primer 4. 5'-GTAGTATCAACAACggatgT**T**A**A**AAA-3' (26mer) (SEQ ID NO: 8)
Primer 5. 5'-GTAGTATCAACAACggatgTAA**T**AAA-3' (26mer) (SEQ ID NO: 9)
Primer 6. 5'-GTAGTATCAACAACggatgTAA**G**AAA-3' (26mer) SEQ ID NO: 10)

Based on HPV 16 DNA template, the primer 1 of the forward primers is the amplification primer (AP) and the primer 2 of the forward primers is the trigger primer (TP).

In addition, the primer 3 of the reverse primers is the amplification primer (AP) and the primers 4-6 of the reverse primers are the trigger primers (TP).

The forward primers were designed in such a manner that 5 bases of each 3' end sequence of them are complementary to the HPV DNA template. The reverse primers were designed in such a manner that 7 bases of each 3' end sequence of them are complementary to the HPV DNA template.

The sequences indicated in lowercase are the restriction enzyme recognition sequences of FokI and BtsCI. In this example, the restriction enzyme recognition sequences were inserted to perform the RFMP assay.

For example, the primer 1 of the forward amplification primer (AP) was designed in such a manner that 5 bases of its 3' end sequence are completely matched with almost all HPV genotypes.

The primer 2 is the forward trigger primer (TP) specially designed to be specific to the HPV 59 DNA because the HPV 59 DNA template is not complementary to and mismatched with the primer 1 of the forward amplification primer (AP) in at least four successive nucleotides (indicated in bold letters).

The primer 3 of the reverse amplification primer (AP) was designed in such a manner that 7 bases of its 3' end sequence are completely matched with the HPV 16 DNA template and bind complementarily to the templates of all HPV genotypes with allowing mismatch of up to three successive bases or 1 to 2 bases.

The primer 4 is the reverse trigger primer (TP), which is complementary to HPV 34, HPV 59 and HPV 73, specifically designed to give variations of the 4^{th} base (A) and the 6^{th} base (T) different from the primer 3 in the 3' end sequence (indicated in bold letters).

The primer 5 is the reverse trigger primer (TP), which is complementary to HPV 51, HPV 70 and HPV 82, specifically designed to give variations of the 4^{th} base (T) different from the primer 3 in the 3' end sequence (indicated in bold letters).

The primer 6 is the reverse trigger primer (TP), which is complementary to HPV 39 and HPV 68, specifically designed to give variations of the 4^{th} base (G) different from the primer 3 in the 3' end sequence (indicated in bold letters).

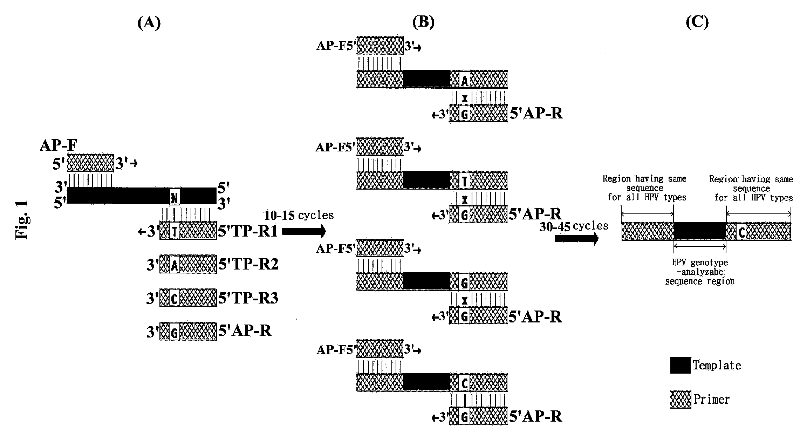

PCR buffer (1x), 2 mM MgSO₄, 200 mM dNTP, 0.5U platinum Taq polymerase (Invitrogen, 10966-026), 0.4 µM primer 1, 0.4 µM primer 3, 0.05 µM primer 2, 0.05 µM primer 4, 0.025 µM primer 5, 0.025 µM primer 6 and 100 ng HPV DNA subjected to be analyzed were added to set 25 µℓ of a total reaction volume. The PCR reaction was performed with the following conditions (see Fig 1. (A) step):
94 °C 5 min;
94 °C 15 second, 45 °C 15 second, 72 °C 15 second (45 cycles); and
72 °C 5 min.

The trigger primers (TP) were bound complementarily to each HPV template to perform a PCR reaction and to produce the PCR amplification products in the initial PCR reaction (see Fig 1. (A) step). At this time, the primers 2 and 4 were bound to the HPV 59 DNA template, the primers 1 and 5 were bound to the HPV 51 DNA template, and the primers 1 and 6 were bound to the HPV 68 DNA template to generate the following amplification products.

From the initial reaction, the primers 1 and 3 of the amplification primers (AP) were bound to the HPV 16 DNA template to generate the following amplification products.

The sequences of the HPV DNA fragments (5' → 3') produced by the initial PCR reaction (10-15 cycles) from each of the HPV DNA templates are represented as follows:
**HPV 59**
**HPV 16**
**HPV 51**
**HPV 68**

Meanwhile, the specific trigger primers (TP) were bound to each of the HPV DNA templates to amplify the templates (in the case of HPV 16 DNA template, the amplification primer (AP) acts as the trigger primer (TP) to bind with the HPV 16 DNA template).

In the PCR amplification process, after the trigger primers (TP) added at a low concentration in the initial stage were completely exhausted and each of the HPV DNA fragments was amplified at a certain concentration, the amplification primers (AP) different from the trigger primers (TP) in a part of the primer-binding sequence of each HPV DNA fragment were bound to the each HPV DNA fragment to perform the amplification (see Fig 1. (B) step).

It is possible because the primer 3 of the reverse amplification primer (AP) was initially designed in such a manner that 7 bases of its 3' end sequence bind complementarily to the templates of all HPV genotypes with allowing mismatch of up to three successive bases or 1 to 2 bases, and was added at a higher concentration than the trigger primers (TP) in the PCR amplification.

According to the present embodiment, only "genotype-analyzable sequences" were replicated from the various HPV DNA templates, and the other sequences (the sequences except for sequences indicated in square brackets ([ ]) in the following sequences) were replicated from the amplification primers (AP) to have the same nucleotide sequences to each other except for the genotype-analyzable sequences (see Fig 1. (C) step).

The final amplification products consist of the majority of the products amplified from the amplification primers (AP), and the minority of the products amplified from the trigger primers (TP) that do not affect the results of analysis.

The sequences of the final HPV DNA fragments (5' → 3') produced from each of the HPV DNA templates are represented as follows:
**HPV 59**
**HPV 16**
**HPV 51**
**HPV 68**

In the sequences produced by the PCR reaction, the sequences represented in lowercase are the restriction enzymes recognition sequences, the underlined sequences are the fragments sequences produced from excision by the restriction enzymes, and the sequences indicated in square brackets ([ ]) are the genotype-analyzable sequences.

In the present embodiment, the PCR amplification was performed only for HPV 59, HPV 16, HPV 51 and HPV 68 using the trigger primers (TP) and the amplification primers (AP), however, it is easily understood by the ordinary skilled person in the art that the PCR amplification using the trigger primers (TP) and the amplification primers (AP) can be applied to the other types of high-risk HPV types and/or low-risk HPV types as mentioned above.

### 2. Excision of restriction enzymes, purification and desalting

1 U FokI (NEB R109L), 1 U BtsCI (NEB R0647S), 50 mM potassium acetate, 20 mM Tris-acetate, 10 mM magnesium acetate and 1 mM DTT (at 25 °C, pH 7.9) were added to the products amplified by the PCR reaction, and reacted for 1 hour at 37°C.

It is desirable to separate the excised DNA fragments from the solution treated with the restriction enzymes and then to measure the molecular weights of the separated DNA fragments.

For example, Oasis (Waters) C18 ion resin exchange column chromatography (C18 reverse phase column chromatography) can be used to separate the DNA fragments excised by the restriction enzymes.

First, 70 µℓ of 0.15M triethyl ammonium acetate (TEAA, pH 7.6) was added to the solution and the mixed solution was maintained for one minute. The resin was activated by flowing 100% acetonitrile (ACN; Sigma, USA) and 1 mℓ of 0.1M TEAA through the column. 100 µℓ of the mixture of the solution treated with the restriction enzymes and 0.15M TEAA solution, 2 mℓ of 0.1M TEAA and 1 mℓ of tertiary distilled water were completely passed through in order.

The column was positioned on the collection plate and 100 µℓ of 70% ACN was put and passed through. The eluate was collected in the collection plate and the collection plate was dried for 60 minutes at 120°C.

### 3. MALDI-TOF Mass Spectrometry

4µℓ of MALDI matrix(22.8 mg of ammonium citrate, 148.5 mg of hydroxypicolinic acid, 1.12 ml of acetonitrile, 7.8 mℓ of H₂O) was previously dotted on the anchor chip plate of MALDI-TOF Mass Spectrometry (Biflex IV, Bruker) and then dried for 30 minutes at 37°C.

10 µℓ of tertiary distilled water was added to the sample of the collection plate obtained through the purification and desalting, and the sample was dissolved out thereby. 2 µℓ of the dissolved solution was dripped dropwise onto the dried MALDI matrix and the MALDI matrix was dried again for 30 minutes at 37°C to perform MALDI-TOF Mass Spectrometry analysis.

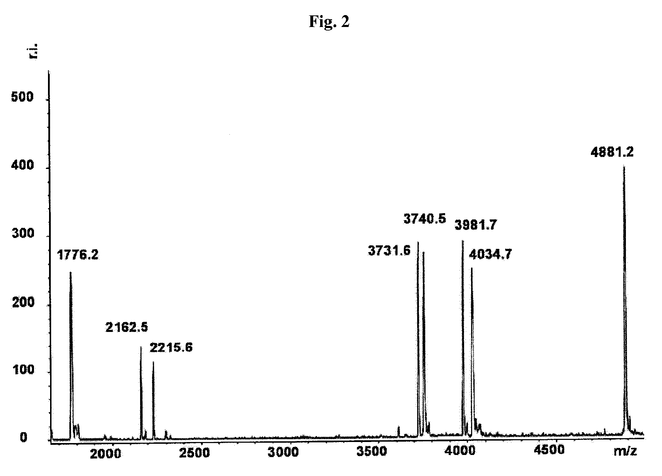

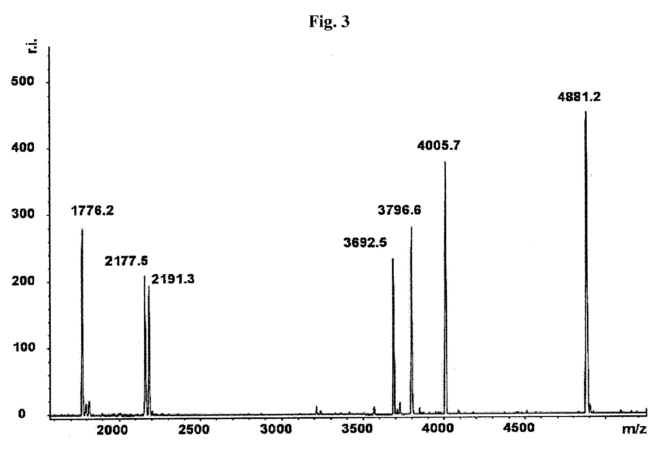

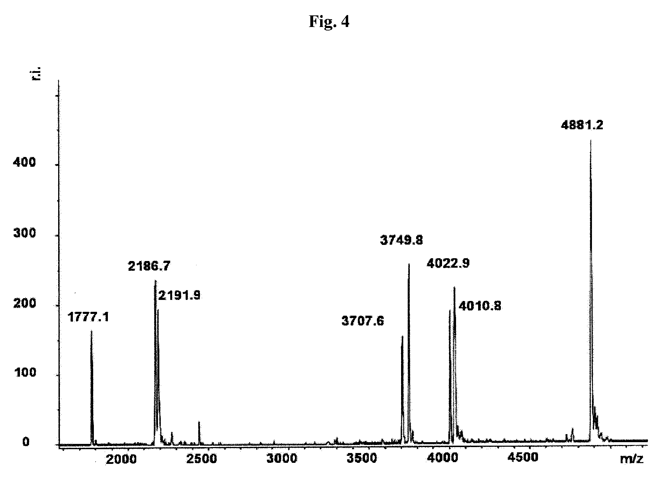

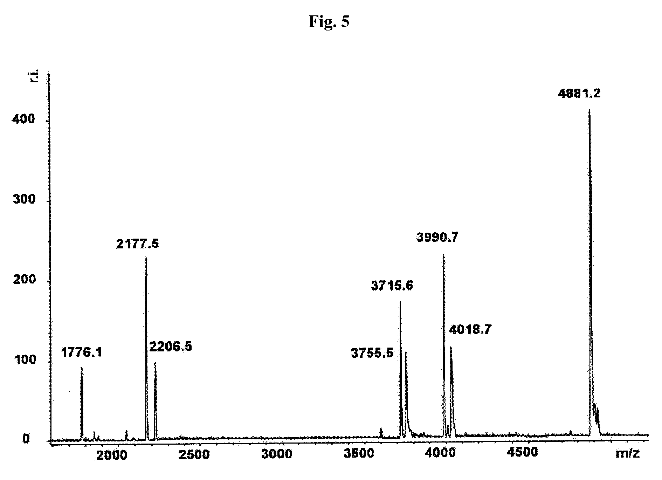

The analysis was performed in accordance with the manual of MALDI-TOF Mass Spectrometry. The analyzed results of the molecular weights of fragments produced by the reaction were illustrated in Figs. 2-5 based on MALDI-TOF Mass Spectrometry. The following table 1 summarizes the results of the analysis on a variety of HPVs as their molecular weights. It is possible to determine the genotypes of HPVs on the basis of the results of table 1.

The table 1 shows the molecular weights of the fragments calculated based on the products amplified by the amplification primers (AP). They differ from the molecular weights of the fragments calculated based on the products amplified by the trigger primers (TP).

According to the embodiment of the present invention, it was confirmed that the molecular weights of the fragments from the amplification primers (AP) and the molecular weights of the fragments from the trigger primers (TP) appeared at the same time when each trigger primer (TP) was mixed at more than a reaction concentration.

On the other hand, it is not possible to perform the analysis of genotypes of the HPVs when each trigger primer (TP) was mixed at less than a reaction concentration.

When the MALDI-TOF Mass Spectrometry graphs for HPV 59, HPV 16, HPV 51 and HPV 68 genotypes were analyzed as shown in Figs. 2-5, the molecular weights of the fragments only from the amplification primers (AP) were confirmed in the amplification products according to the embodiment of the present invention.

Therefore, if the RFMP assay is performed after PCR amplification using the trigger primers (TP) and the amplification primers (AP) of the present invention, it is possible to amplify a number of variant genes simply and efficiently without missing and to analyze a number of genotypes precisely with satisfying both specificity and sensitivity.

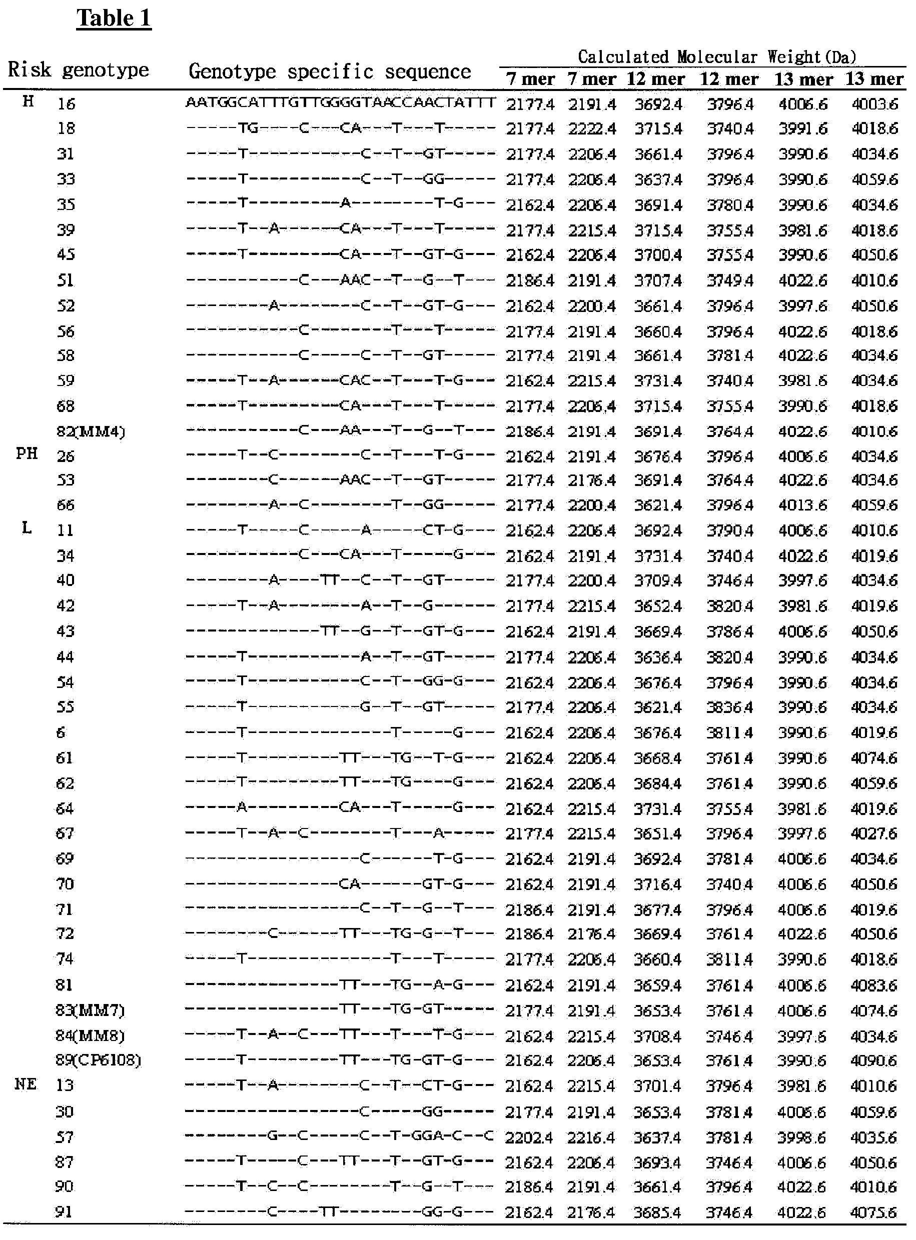

### Example 2. HLA(Human Leukocyte Antigen)-DQB1 genotyping

It was further confirmed whether the PCR amplification using the trigger primers (TP) and the amplification primers (AP) of the present invention can be applied to the analysis of the various genotypes. For example, the genotyping of HLA (Human Leukocyte Antigen)-DQB1 alleles associated with the human immune response was performed by the RFMP assay after the PCR amplification using the trigger primers (TP) and the amplification primers (AP). Example 2 was performed for human genomic DNAs.

### 1. PCR amplification

A pair of amplification primers (AP) were prepared by aligning about 100 HLA-DQB1 sequences to determine "genotype-analyzable sequences" and by selecting a primer-binding portion.

In addition, a pair of trigger primers (TP) were prepared to bind complementarily to HLA-DQB1 *0201 DNA template for the specific sequences of the 3' and 5' ends of the HLA-DQB1 *0201 type.

The PCR was carried out using a mixture including a high concentration of the amplification primers (AP) and a low concentration of trigger primers (TP) as designed above. The template DNA sequences (5' → 3') amplified by the primers in the HLA-DQB1 alleles subjected to be analyzed are represented as follows:
**HLA-DQB1 *0501**
**HLA-DQB1 *0201**
**HLA-DQB1 *0401**
**HLA-DQB1 *0301**

The underlined sequences of the template DNA sequences of the HLA-DQB1 alleles are binding portions of the primer Nos. 7-10 as below. The underlined sequence in front of the template DNA sequence of each HLA-DQB1 allele is a binding portion of a forward primer. The underlined sequence in the rear of the template DNA sequence of each HLA-DQB 1 allele is a binding portion of a reverse primer. The sequence represented in lowercase is the genotype-analyzable sequence.
Primer 7. 5'-TGTGCTACTTCACCAACggatgGACGGAGC-3' (30mer) (SEQ ID NO: 23);
Primer 8. 5'-GCGTGCGTACTCCTCTCGGTggatgATAGATGT-3' (33mer) (SEQ ID NO: 24);
Primer 9. 5'-TGTGCTACTTCACCAACggatgG**A**CAGAGC-3' (30mer) (SEQ ID NO: 25); and
Primer 10. 5'-GCGCACGATCTCTTCTCGGTggatgATAGATGC-3' (33mer) (SEQ ID NO: 26).

The primer 7 of the forward primers is the amplification primer (AP) and the primer 9 of the forward primers is the trigger primer (TP). In addition, the primer 8 of the reverse primers is the amplification primer (AP) and the primer 10 of the reverse primers is the trigger primer (TP).

The forward primers were designed in such a manner that 4 bases of each 3' end sequence of them are complementary to the HLA-DQB1 DNA template. The reverse primers were designed in such a manner that 7 bases of each 3' end sequence of them are complementary to the HLA-DQB1 DNA template.

The sequences indicated in lowercase are the restriction enzyme recognition sequences of FokI and BtsCI. In this example, the restriction enzyme recognition sequences were inserted to perform the RFMP assay.

For example, the primer 7 of the forward amplification primer (AP) was designed in such a manner that 4 bases of its 3' end sequence are completely matched with almost all HLA-DQB1 genotypes. The primer 9 is the forward trigger primer (TP) specially designed to be specific to the HLA-DQB1 *0201 because the HLA-DQB1 *0201 DNA template is not complementary to and mismatched with the primer 7 of the forward amplification primer (AP) in one nucleotide (A; indicated in bold letters).

The primer 8 of the reverse amplification primer (AP) was designed in such a manner that 7 bases of its 3' end sequence are completely matched with all HLA-DQB1 genotypes except for HLA-DQB1 *0201.

The primer 10 is the reverse trigger primer (TP), which is complementary to HLA-DQB1 *0201, designed for its one nucleotide of 3' end sequence and its five nucleotides of 5' end sequence to be specifically matched with the template of the HLA-DQB1 *0201.

PCR buffer (1x), 2 mM MgSO₄, 200 mM dNTP, 0.5U platinum Taq polymerase (Invitrogen, 10966-026), 0.4 µM primer 7, 0.4 µM primer 8, 0.025 µM primer 9, 0.025 µM primer 10 and 100 ng human DNA were added to set 25 µℓ of a total reaction volume. The PCR reaction was performed with the following conditions (see Fig 1. (A) step).
94°C 5 min;
94°C 15 second, 45°C 15 second, 72 °C 15 second (45 cycles); and
72°C 5 min.

The trigger primers (TP) were bound complementarily to each HLA-DQB1 template to perform a PCR reaction and to produce the PCR amplification products in the initial PCR reaction (see Fig 1. (A) step).

For example, the primers 9 and 10 were bound to the HLA-DQB1 *0201 DNA template to generate the following amplification products. The sequence of the HLA-DQB1 *0201 DNA fragment (5' → 3') produced by the initial PCR reaction (10-15 cycles) from the HLA-DQB1 *0201 DNA template is represented as follows:
**HLA-DQB1 *0201**

Meanwhile, the specific trigger primers (TP) were bound to the HLA-DQB1 *0201 DNA template to amplify the template.

In the PCR amplification process, after the trigger primers (TP) added at a low concentration in the initial stage were completely exhausted and each of the HLA-DQB1 DNA fragments was amplified at a certain concentration, the amplification primers (AP) different from the trigger primers (TP) in a part of the primer-binding sequence of each HLA-DQB1 DNA fragment were bound to each HLA-DQB1 DNA fragment to perform the amplification (see Fig 1. (B) step).

It is possible because the primer 8 of the amplification primer (AP) was initially designed in such a manner that 7 bases of its 3' end sequence bind complementarily to all HLA-DQB1 genotypes with allowing mismatch of up to three successive bases or 1 to 2 bases, and was added at a higher concentration than the trigger primers (TP) in the PCR amplification.

According to the present embodiment, only "genotype-analyzable sequences" were replicated from the various HLA-DQB1 DNA templates, and the other sequences (the sequences except for sequences indicated in square brackets ([ ]) in the following sequences) were replicated from the amplification primers (AP) to have the same nucleotide sequences to each other except for the genotype-analyzable sequences (see Fig 1. (C) step).

The final amplification products consist of the majority of the products amplified from the amplification primers (AP), and the minority of the products amplified from the trigger primers (TP) that do not affect the results of analysis.

The sequences of the final HLA-DQB1 DNA fragments (5' → 3') produced from each of the HLA-DQB1 DNA templates are represented as follows:
**HLA-DQB1 *0501**
**HLA-DQB1 *0201**
**HLA-DQB1 *0401**
**HLA-DQB1 *0301**

In the sequences produced by the PCR reaction, the sequences represented in lowercase are the restriction enzymes recognition sequences, the underlined sequences are the fragments sequences produced from excision by the restriction enzymes, and the sequences indicated in square brackets ([ ]) are the genotype-analyzable sequences. The principle for generating the amplified products is the same as that of Example 1.

In the present embodiment, the PCR amplification was performed only for HLA-DQB1 *0501, HLA-DQB1 *0201, HLA-DQB1 *0401, HLA-DQB1 *0301 using the trigger primers (TP) and the amplification primers (AP), however, it is easily understood by the ordinary skilled person in the art that the PCR amplification using the trigger primers (TP) and the amplification primers (AP) can be applied to the other allele types of HLA-DQB1 1 types as mentioned above.

### 2. Excision of restriction enzymes, purification and desalting

The procedures were performed in the same manner as in Example 1.

### 3. MALDI-TOF Mass Spectrometry

The procedures were performed in the same manner as in Example 1.

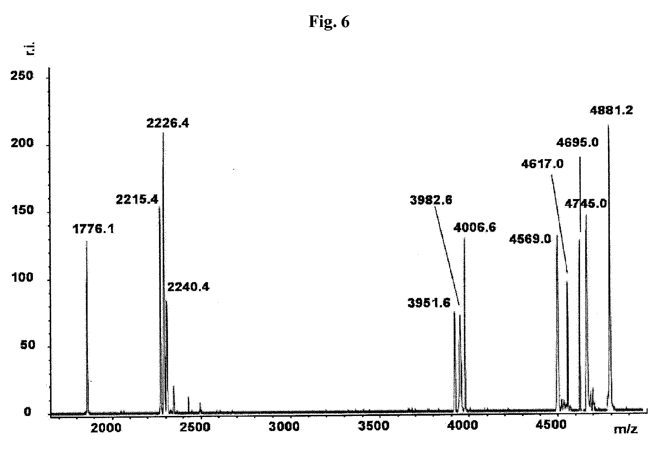

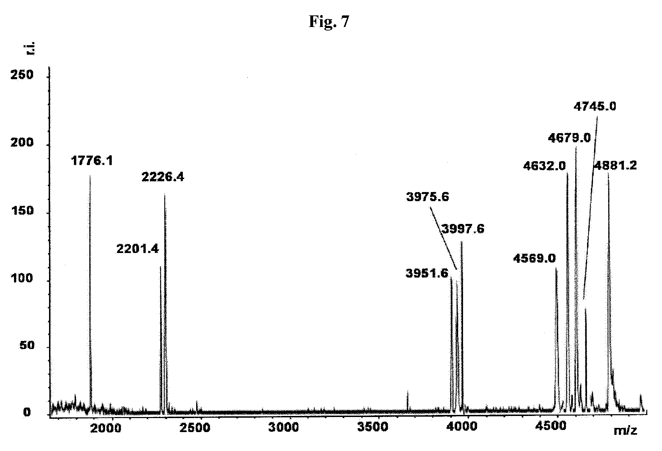

The analysis was performed in accordance with the manual of MALDI-TOF Mass Spectrometry. The analyzed results of the molecular weights of fragments produced by the reaction were illustrated in Figs. 6-7 based on MALDI-TOF Mass Spectrometry. The following table 2 summarizes the results of the analysis on a variety of HLA-DQB1 alleles as their molecular weights. It is possible to determine the genotypes of HLA-DQB1 alleles on the basis of the results of table 2.

The table 2 shows the molecular weights of the fragments calculated based on the products amplified by the amplification primers (AP). They differ from the molecular weights of the fragments calculated based on the products amplified by the trigger primers (TP).

According to the embodiment of the present invention, it was confirmed that the molecular weights of the fragments from the amplification primers (AP) and the molecular weights of the fragments from the trigger primers (TP) appeared at the same time when the trigger primers (TP) were mixed at more than 0.025 µM.

On the other hand, it is not possible to perform the analysis of genotypes of the HLA-DUB1 alleles when the trigger primers (TP) were mixed at less than 0.025 µM.

Figs. 6-7 show a graph of MALDI-TOF mass spectrometry for human hetero-genotype of HLA-DQB1 (0201/0501) and a graph of MALDI-TOF mass spectrometry for human hetero-genotype of HLA-DQB1 (0301/0401), respectively. From the Figs. 6-7, the molecular weights of the fragments only from the amplification primers (AP) were confirmed in the amplification products according to the embodiment of the present invention.

Therefore, if the RFMP assay is performed after PCR amplification using the trigger primers (TP) and the amplification primers (AP) of the present invention, it is possible to amplify a number of variant genes simply and efficiently without missing and to analyze a number of genotypes precisely with satisfying both specificity and sensitivity.

**Table 2**

| **HLA**-**DQB1** | **HLA-DQ** Serological specificities | Calculated Molecular Weight **(Da)** | | | | | |
|---|---|---|---|---|---|---|---|
| | | **F** | **R** | **UP** | **DW** | **F** | **R** |
| Alleles | | 7 **mer** | **13mer** | **15mer** | **15mer** | **13mer** | **7mer** |
| DQB1*050101 | DQ5 | 2226.4 | 3951.6 | 4745.0 | 4569.0 | 3982.6 | 2240.4 |
| DQB1*050102 | DQ5 | 2226.4 | 3951.6 | 4745.0 | 4569.0 | 3982.6 | 2240.4 |
| DQB1*050302 | DQ5 | 2226.4 | 3951.6 | 4745.0 | 4569.0 | 3982.6 | 2240.4 |
| DQB1*030201 | DQ8 | 2226.4 | 3951.6 | 4655.0 | 4657.0 | 3997.6 | 2224.4 |
| DQB1*030203 | DQ8 | 2226.4 | 3951.6 | 4655.0 | 4657.0 | 3997.6 | 2224.4 |
| DQB1*030204 | DQ8 | 2226.4 | 3951.6 | 4655.0 | 4657.0 | 3997.6 | 2224.4 |
| DQB1*0307 | DQ8 | 2226.4 | 3951.6 | 4655.0 | 4657.0 | 3997.6 | 2224.4 |
| DQB1*0311 | DQ8 | 2226.4 | 3951.6 | 4655.0 | 4657.0 | 3997.6 | 2224.4 |
| DQB1*030202 | DQ8 | 2226.4 | 3951.6 | 4655.0 | 4657.0 | 3997.6 | 2224.4 |
| DQB1*0308 | DQ8 | 2226.4 | 3951.6 | 4655.0 | 4657.0 | 3997.6 | 2224.4 |
| DQB1*030302 | DQ9 | 2226.4 | 3951.6 | 4655.0 | 4657.0 | 3997.6 | 2224.4 |
| DQB1*030303 | DQ9 | 2226.4 | 3951.6 | 4655.0 | 4657.0 | 3997.6 | 2224.4 |
| DQB1*020101 | DQ2 | 2226.4 | 3951.6 | 4695.0 | 4617.0 | 4006.6 | 2215.4 |
| DQB1*020102 | DQ2 | 2226.4 | 3951.6 | 4695.0 | 4617.0 | 4006.6 | 2215.4 |
| DQB1*0202 | DQ2 | 2226.4 | 3951.6 | 4695.0 | 4617.0 | 4006.6 | 2215.4 |
| DQB1*0204 | DQ2 | 2226.4 | 3951.6 | 4695.0 | 4617.0 | 4006.6 | 2215.4 |
| DQB1*0205 | DQ2 | 2226.4 | 3951.6 | 4695.0 | 4617.0 | 4006.6 | 2215.4 |
| DQB1*0203 | DQ2 | 2226.4 | 3951.6 | 4695.0 | 4617.0 | 4006.6 | 2215.4 |
| DQB1*0314 | DQ3 | 2226.4 | 3951.6 | 4679.0 | 4632.0 | 3997.6 | 2224.4 |
| DQB1*0304 | DQ7 | 2226.4 | 3951.6 | 4679.0 | 4632.0 | 3997.6 | 2224.4 |
| DQB1*0310 | DQ3 | 2226.4 | 3951.6 | 4679.0 | 4632.0 | 3997.6 | 2224.4 |
| DQB1*030101 | DQ7 | 2226.4 | 3951.6 | 4679.0 | 4632.0 | 3997.6 | 2224.4 |
| DQB1*030102 | DQ7 | 2226.4 | 3951.6 | 4679.0 | 4632.0 | 3997.6 | 2224.4 |
| DQB1*030104 | DQ7 | 2226.4 | 3951.6 | 4679.0 | 4632.0 | 3997.6 | 2224.4 |
| DQB1*030103 | DQ7 | 2226.4 | 3951.6 | 4679.0 | 4632.0 | 3997.6 | 2224.4 |
| DQB1*0316 | DQ7 | 2226.4 | 3951.6 | 4679.0 | 4632.0 | 3997.6 | 2224.4 |
| DQB1*060101 | DQ6 | 2226.4 | 3951.6 | 4679.0 | 4632.0 | 3997.6 | 2224.4 |
| DQB1*060102 | DQ6 | 2226.4 | 3951.6 | 4679.0 | 4632.0 | 3997.6 | 2224.4 |
| DQB1*060103 | DQ6 | 2226.4 | 3951.6 | 4679.0 | 4632.0 | 3997.6 | 2224.4 |
| DQB1*060104 | DQ6 | 2226.4 | 3951.6 | 4679.0 | 4632.0 | 3997.6 | 2224.4 |
| DQB1*0635 | DQ6 | 2226.4 | 3951.6 | 4679.0 | 4632.0 | 3997.6 | 2224.4 |
| DQB1*040101 | DQ4 | 2201.4 | 3975.6 | 4745.0 | 4569.0 | 3997.6 | 2224.4 |
| DQB1*040102 | DQ4 | 2201.4 | 3975.6 | 4745.0 | 4569.0 | 3997.6 | 2224.4 |

### Example 3. Analysis of base variations of hepatitis B virus DNA polymerase related to Adefovir-resistance

It occasionally occurs that there are base variations in the DNA polymerase gene of hepatitis B virus that causes human hepatitis type B. The resistance to Adefovir for treating hepatitis type B occurs by some base variations. It has been reported that the mutations of the amino acids of A181V/T and N236T casuse the resistance to Adefovir.

It was further confirmed whether the PCR amplification using the trigger primers (TP) and the amplification primers (AP) of the present invention can be applied to the analysis of the various genotypes. In other words, the genotyping of hepatitis B virus having the resistance to Adefovir was performed by the RFMP assay after the PCR amplification using the trigger primers (TP) and the amplification primers (AP). Example 3 was performed for HBV DNAs.

### 1. PCR amplification

A pair of amplification primers (AP) were prepared to analyze the nucleotides of the 236^{th} amino acid in the DNA polymerase gene of hepatitis B virus. In addition, several trigger primers (TP), each of which is specific to a variety of HBV DNA templates, were prepared with regard to the nucleotide sequences of the 237^{th} and 238^{th} amino acids having polymorphisms. The PCR was carried out using a mixture including a high concentration of the amplification primers (AP) and a low concentration of trigger primers (TP) as designed above. The template DNA sequences (5' → 3') amplified by the primers in the HBV genes subjected to be analyzed are represented as follows:
**HBV #** 1 **(General type)**
**HBV** # **2**
**HBV # 3**
**HBV # 4**
**HBV # 5**
**HBV # 6**
**HBV # 7**
**HBV # 8**

The underlined sequences of the HBV DNA template sequences are binding portions of the primer Nos. 11-19 as below. The underlined sequence in front of each HBV DNA template is a binding portion of a forward primer. The underlined sequence in the rear of each HBV DNA template is a binding portion of a reverse primer. The sequence represented in lowercase is the genotype-analyzable sequence.

### * Forward primer

Primer 11.
5'-TACCAATTTTCTTTTggatgTGTCTTTGGGTATACATTT-3' (39mer) (SEQ ID NO: 40)

### * Reverse primers

Primer 12. 5'-CCCAACGTTTggatgGGTTTTATTAGG-3' (27mer) (SEQ ID NO: 41)
Primer 13. 5'-CCCAACGTTTggatgGGTTTTA**G**TAGG-3' (27mer) (SEQ ID NO: 42)
Primer 14. 5'-CCCAACGTTTggatgGGTTTTAT**C**AGG-3' (27mer) (SEQ ID NO: 43)
Primer 15. 5'-CCCAACGTTTggatgGGTTTTAT**G**AGG-3' (27mer) (SEQ ID NO: 44)
Primer 16. 5'-CCCAACGTTTggatgGGTTTTATTA**T**G-3' (27mer) (SEQ ID NO: 45)
Primer 17. 5'-CCCAACGTTTggatgGGTTTTA**G**TA**T**G-3' (27mer) (SEQ ID NO: 46)
Primer 18. 5'-CCCAACGTTTggatgGGTTTTAT**C**A**T**G-3' (27mer) (SEQ ID NO: 47)
Primer 19. 5'-CCCAACGTTTggatgGGTTTTAT**G**A**T**G-3' (27mer) (SEQ ID NO: 48)

Based on HBV #1 DNA template, the primer 11 of the forward primer is the amplification primer (AP), the primer 12 of the reverse primers is the amplification primer (AP), and the primers 13-19 of the reverse primers are the trigger primers (TP). The forward primer was designed in such a manner that 19 bases of its 3' end sequence are complementary to the HBV DNA template. The reverse primers were designed in such a manner that 12 bases of each 3' end sequence of them are complementary to the HBV DNA template. The sequences indicated in lowercase are the restriction enzyme recognition sequences of FokI. In this example, the restriction enzyme recognition sequences were inserted to perform the RFMP assay.

For example, the primer 11 of the forward amplification primer (AP) was designed in such a manner that 19 bases of its 3' end sequence are matched with all HBV genotypes. The primer 12 of the reverse amplification primer (AP) was designed in such a manner that 12 bases of its 3' end sequence are completely matched with HBV #1 DNA template and bind complementarily to the templates of all HBV genotypes with allowing mismatch of up to three successive bases or 1 to 2 bases.

For designing the trigger primers, it was considered that the variant HBVs have nucleotide sequences different from the general type-HBV in the 3'end of the HBV DNA. The primer Nos. 13-19, each of which is specific to each of the variant HBV DNA templates, were prepared with regard to the nucleotide sequences of the 237^{th} and 238^{th} amino acids. The primer 13 is the reverse trigger primer (TP), which is complementary to HBV #2, specifically designed to give a variation of the 5^{th} base (G) different from the primer 12 in the 3' end sequence (indicated in bold letters). The primer 14 is the reverse trigger primer (TP), which is complementary to HBV #3, specifically designed to give a variation of the 4^{th} base (C) different from the primer 12 in the 3' end sequence (indicated in bold letters). The primer 15 is the reverse trigger primer (TP), which is complementary to HBV #4, specifically designed to give a variation of the 4^{th} base (G) different from the primer 12 in the 3' end sequence (indicated in bold letters). The primer 16 is the reverse trigger primer (TP), which is complementary to HBV #5, specifically designed to give a variation of the 2nd base (T) different from the primer 12 in the 3' end sequence (indicated in bold letters). The primer 17 is the reverse trigger primer (TP), which is complementary to HBV #6, specifically designed to give variations of the 2nd base (T) and the 5^{th} base (G) different from the primer 12 in the 3' end sequence (indicated in bold letters). The primer 18 is the reverse trigger primer (TP), which is complementary to HBV #7, specifically designed to give variations of the 2nd base (T) and the 4^{th} base (C) different from the primer 12 in the 3' end sequence (indicated in bold letters). Finally, the primer 19 is the reverse trigger primer (TP), which is complementary to HBV #8, specifically designed to give variations of the 2nd base (T) and the 4^{th} base (G) different from the primer 12 in the 3' end sequence (indicated in bold letters).

PCR buffer (1x), 2 mM MgSO₄, 200 mM dNTP, 0.5U platinum Taq polymerase (Invitrogen, 10966-026), 0.4 µM primer 11, 0.4 µM primer 12, 0.07 µM primer 13, 0.07 µM primer 14, 0.04 µM primer 15, 0.04 µM primer 16, 0.05 µM primer 17, 0.05 µM primer 18, 0.05 µM primer 19 and 100 ng HBV DNA subjected to be analyzed were added to set 25 µℓ of a total reaction volume. The PCR reaction was performed with the following conditions (see Fig 1. (A) step).
94°C 5 min;
94°C 15 second, 45°C 15 second, 72 °C 15 second (45 cycles); and
72 °C 5 min.

The trigger primers (TP) were bound complementarily to each HBV template to perform a PCR reaction and to produce the PCR amplification products in the initial PCR reaction (see Fig 1. (A) step). At this time, the primers 11 and 13 were bound to the HBV #2 DNA template, the primers 11 and 14 were bound to the HBV #3 DNA template, the primers 11 and 15 were bound to the HBV #4 DNA template, the primers 11 and 16 were bound to the HBV #5 DNA template, the primers 11 and 17 were bound to the HBV #6 DNA template, the primers 11 and 18 were bound to the HBV #7 DNA template, and the primers 11 and 19 were bound to the HBV #8 DNA template to generate the following amplification products. From the initial reaction, the primers 11 and 12 of the amplification primers (AP) were bound to the HBV #1 DNA template to generate the following amplification products. The sequences of the HBV DNA fragments (5' → 3') produced by the initial PCR reaction (10-15 cycles) from each of the HBV DNA templates are represented as follows:
**HBV # 1 (General type)**
**HBV** # **2**
**HBV # 3**
**HBV # 4**
**HBV # 5**
**HBV # 6**
**HBV # 7**
**HBV # 8**

Meanwhile, the specific trigger primers (TP) were bound to each of the HBV DNA templates to amplify the templates (in the case of HBV #1 DNA template, the amplification primer (AP) acts as the trigger primer (TP) to bind with the HBV #1 DNA template). In the PCR amplification process, after the trigger primers (TP) added at a low concentration in the initial stage were completely exhausted and each of the HBV DNA fragments was amplified at a certain concentration, the amplification primers (AP) different from the trigger primers (TP) in a part of the primer-binding sequence of each HBV DNA fragment were bound to the each HBV DNA fragment to perform the amplification (see Fig 1. (B) step).

It is possible because the primer 12 of the reverse amplification primer (AP) was initially designed in such a manner that 12 bases of its 3' end sequence bind complementarily to the templates of all HBV genotypes with allowing mismatch of up to three successive bases or 1 to 2 bases, and was added at a higher concentration than the trigger primers (TP) in the PCR amplification.

According to the present embodiment, only "genotype-analyzable sequences" were replicated from the various HBV DNA templates, and the other sequences (the sequences except for sequences indicated in square brackets ([ ]) in the following sequences) were replicated from the amplification primers (AP) to have the same nucleotide sequences to each other except for the genotype-analyzable sequences (see Fig 1. (C) step). The final amplification products consist of the majority of the products amplified from the amplification primers (AP), and the minority of the products amplified from the trigger primers (TP) that do not affect the results of analysis.

The sequences of the final HBV DNA fragments (5' → 3') produced from each of the HBV DNA templates are represented as follows:
**HBV # 1 (General type)**
**HBV** # **2**
**HBV # 3**
**HBV # 4**
**HBV # 5**
**HBV # 6**
**HBV # 7**
**HBV # 8**

In the sequences produced by the PCR reaction, the sequences represented in lowercase are the restriction enzymes recognition sequences, the underlined sequences are the fragments sequences produced from excision by the restriction enzymes, and the sequences indicated in square brackets ([ ]) are the genotype-analyzable sequences. The principle for generating the amplified products is the same as that of Example 1.

In the present embodiment, the PCR amplification was performed only for HBV #1 to HBV #8 using the trigger primers (TP) and the amplification primers (AP), however, it is easily understood by the ordinary skilled person in the art that the PCR amplification using the trigger primers (TP) and the amplification primers (AP) can be applied to the other variant HBV types as mentioned above.

### 2. Excision of restriction enzymes, purification and desalting

The procedures were performed in the same manner as in Example 1.

### 3. MALDI-TOF Mass Spectrometry

The procedures were performed in the same manner as in Example 1.

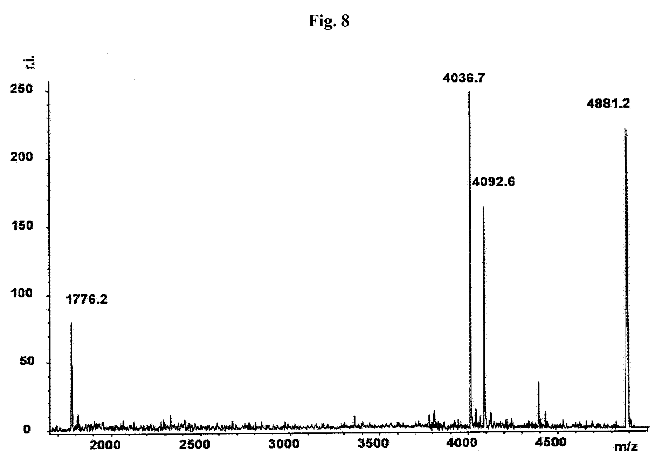

The analyzed results of the molecular weights of fragments produced by the reaction were illustrated in Figs. 8-9 based on MALDI-TOF Mass Spectrometry. The following table 3 summarizes the results of the analysis on a variety of HBVs as their molecular weights. It is possible to determine the genotypes of HBVs on the basis of the results of table 3.

The table 3 shows the molecular weights of the fragments calculated based on the products amplified by the amplification primers (AP). They differ from the molecular weights of the fragments calculated based on the products amplified by the trigger primers (TP).

According to the embodiment of the present invention, it was confirmed that the molecular weights of the fragments from the amplification primers (AP) and the molecular weights of the fragments from the trigger primers (TP) appeared at the same time when each trigger primer (TP) was mixed at more than a reaction concentration.

On the other hand, it is not possible to perform the analysis of genotypes of the HBVs when each trigger primer (TP) was mixed at less than a reaction concentration.

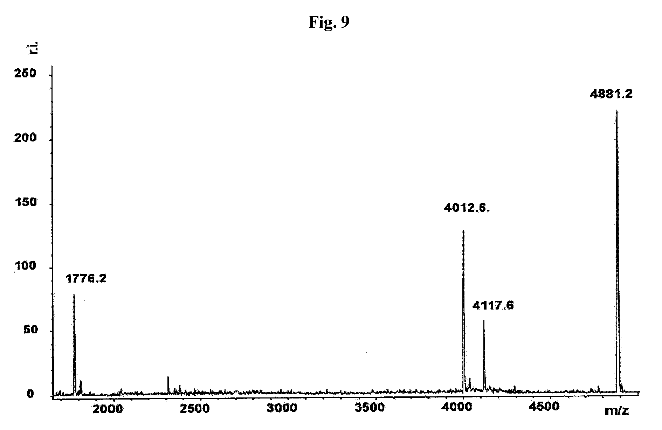

In this regard, a graph of MALDI-TOF mass spectrometry is presented in Fig. 8, in the event that HBV polymerase inducing adefovir-resistance by its base variation shows wild-type HBV genotype where its 236th condon is AAC for coding the amino acid of asparagine (Asn). In addition, a graph of MALDI-TOF mass spectrometry is presented in Fig. 9, in the event that HBV polymerase inducing adefovir-resistance by its base variation shows mutant-type HBV genotype where its 236th condon is ACT for coding the amino acid of threonine (Thr).

When the MALDI-TOF Mass Spectrometry graphs were analyzed as shown in Figs. 8-9, the molecular weights of the fragments only from the amplification primers (AP) were confirmed in the amplification products according to the embodiment of the present invention.

Therefore, if the RFMP assay is performed after PCR amplification using the trigger primers (TP) and the amplification primers (AP) of the present invention, it is possible to amplify a number of variant genes simply and efficiently without missing and to analyze a number of genotypes precisely with satisfying both specificity and sensitivity.

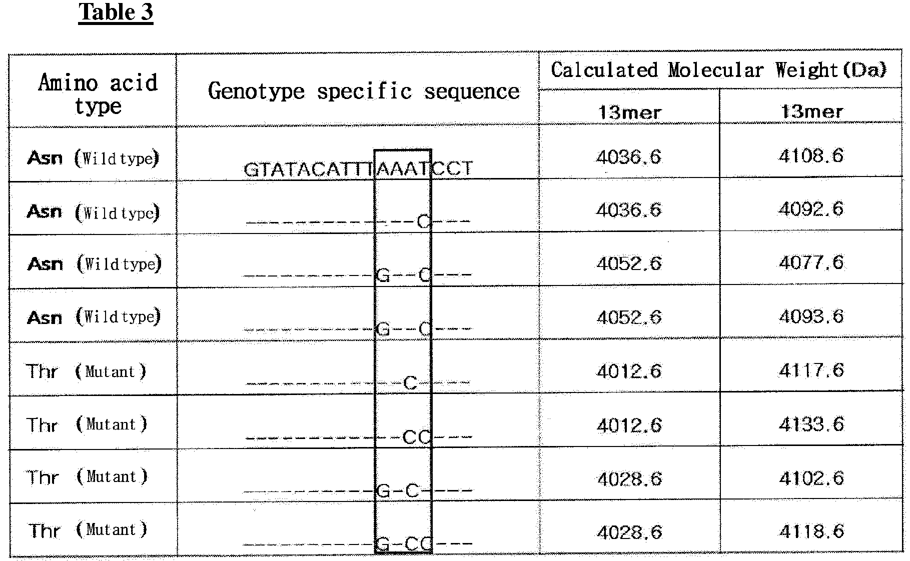

## Claims

1. A method using a primer composition for amplifying a gene region having various variations and a genotype-analyzable sequence in a target gene comprising:
(a) preparing a first primer group comprising a plurality of primers specific to a plurality of templates, respectively, each of the templates includes the genotype-analyzable sequence and each of the primers being specific to a variation in the target gene;
(b) preparing a second primer group comprising a combination of one forward primer any one reverse primer the forward and/or the reverse primer being designed by selecting one primer having the greatest number of shared bases from the primers of the first primer group and by using the selected primer as a reference for the primers of the first primer group to have the same sequence and length as the selected primer, the forward primer and/or the reverse primer of the second primer group binding specifically to one of the templates and binding to the other of the templates with allowing mismatch of up to three successive bases or 1 to 2 bases in the vicinity of the 3' end of the templates, wherein the primers of the first primer group and the second primer group are complementary to a sequence which flanks the genotype-analyzable sequence;
(c) performing a PCR amplification reaction by adding the primers of the first primer group and the combination of the forward primer and the reverse primer of the second primer group to an amplification reactant including at least one polynucleotide template, a DNA polymerase, dNTPs, and a buffer solution, wherein the first primer group is added to the amplification reactent at a low concentration, and the second primer group is added to the amplification reactant at a high concentration as compared with the first primer group;
(d) binding each primer of the first primer group complementarily to the corresponding template, amplifying the template and exhausting the first primer group in the initial PCR amplification reaction;
(e) performing further amplification reaction by the combination of the forward primer and the reverse primer of the second primer group, using the first amplification products amplified by the primers of the first primer group as templates;
(f) obtaining final amplification products each of which has the genotype-analyzable sequence, being amplified to have the same length to each other and to have different nucleotide sequences only in the genotype-analyzable sequence; and
wherein the final amplification products have the same length as the first amplification products.

2. The method as claimed in claim 1, wherein the concentration of the primer of the first primer group is 1/2 to 1/20 of the concentration of the primer of the second primer group in the (c) step.

3. The method as claimed in claim 2, wherein the concentration of the primer of the first primer group is 1/8 to 1/16 of the concentration of the primer of the second primer group in the (c) step.

4. The method as claimed in claim 1, wherein the primer of the first primer group is at least one primer selected from the group consisting of SEQ ID NO: 6 and SEQ ID NOs: 8-10, and the primer of the second primer group is at least one primer selected from the group consisting of SEQ ID NO: 5 and SEQ ID NO: 7 when the target gene is HPV gene.

5. A method for genotyping a target gene using a primer composition for amplifying a gene region having various variations in a target gene comprising:
(a) amplifying the target gene according to the method of any one of claims 1 to 4;
(b) excising a polynucleotide amplified in the (a) step by restriction enzymes, the polynucleotide including a genotype-analyzable sequence of a genetically variant base sequence subjected to be analyzed in the target gene;
(c) generating at least two kinds of single strand polynucleotide fragments having the variant base by the excision of the restriction enzymes, the number of bases of the fragments being within a limited range; and
(d) measuring the molecular weights of the fragments.

6. The method as claimed in claim 5, wherein the number of bases of the fragments ranges between 2 and 32.

7. The method as claimed in claim 5, wherein the primer of the first primer group is at least one primer selected from the group consisting of SEQ ID NO: 6 and SEQ ID NOs: 8-10, and the primer of the second primer group is at least one primer selected from the group consisting of SEQ ID NO: 5 and SEQ ID NO: 7 when the target gene amplified in the (a) step is HPV gene.

## Patentansprüche

1. Verfahren, das eine Primerzusammensetzung verwendet, zur Amplifizierung einer Genregion mit verschiedenen Variationen und einer Genotyp-analysierbaren Sequenz in einem Targetgen, umfassend:
(a) Herstellen einer ersten Primergruppe, die mehrere Primer enthält, die jeweils für mehrere Templates spezifisch sind, wobei jedes der Templates die Genotyp-analysierbare Sequenz enthält und jeder der Primer für eine Variation im Targetgen spezifisch ist;
(b) Herstellen einer zweiten Primergruppe, die eine Kombination aus einem Vorwärtsprimer und einem Rückwärtsprimer enthält, wobei der Vorwärts- und/oder der Rückwärtsprimer durch das Auswählen eines Primers mit der größten Anzahl an gemeinsamen Basen der Primer der ersten Primergruppe und durch das Verwenden des ausgewählten Primers als Referenz für die Primer der ersten Primergruppe, derart gestaltet ist, dass sie dieselbe Sequenz und Länge wie der ausgewählte Primer hat, wobei der Vorwärtsprimer und/oder der Rückwärtsprimer der zweiten Primergruppe spezifisch an eines der Templates bindet und an das andere der Templates bindet, mit dem Erlauben einer falschen Kombination von bis zu drei aufeinanderfolgenden Basen oder 1 bis 2 Basen in der Nähe des 3'-Endes der Template, wobei die Primer der ersten Primergruppe und der zweiten Primergruppe komplementär zu einer Sequenz sind, die an die Genotyp-analysierbare Sequenz angrenzt;
(c) Durchführen einer PCR-Amplifizierungsreaktion durch das Hinzufügen der Primer der ersten Primergruppe und die Kombination des Vorwärtsprimers und des Rückwärtsprimers der zweiten Primergruppe zu einem Amplifizierungsreaktanten einschließlich wenigstens eines Polynukleotidtemplates, einer DNA-Polymerase, dNTPs und einer Pufferlösung, wobei die erste Primergruppe dem Amplifizierungsreaktanten in einer niedrigen Konzentration hinzugefügt wird und die zweite Primergruppe dem Amplizifizierungsreaktanten in einer hohen Konzentration im Vergleich zur ersten Primergruppe hinzugefügt wird;
(d) Binden jedes Primers der ersten Primergruppe komplementär an das entsprechende Template, Amplifizieren des Templates und Verbrauchen der ersten Primergruppe in der Anfangs-PCR-Amplifizierungsreaktion;
(e) Durchführen einer weiteren Amplifizierungsreaktion durch die Kombination des Vorwärtsprimers und des Rückwärtsprimers der zweiten Primergruppe, unter Verwendung der ersten Amplifizierungsprodukte amplifiziert durch die Primer der ersten Primergruppe als Templates;
(f) Erhalten von finalen Amplifizierungsprodukten, von denen jedes die Genotyp-analysierbare Sequenz aufweist, derart amplifiziert, dass sie dieselbe Länge zueinander haben und unterschiedliche Nukleotidsequenzen nur in der Genotyp-analysierbaren Sequenz haben; und
wobei die finalen Amplifizierungsprodukte dieselbe Länge wie die ersten Amplifizierungsprodukte aufweisen.

2. Verfahren nach Anspruch 1, wobei die Konzentration des Primers der ersten Primergruppe 1/2 bis 1/20 der Konzentration des Primers der zweiten Primergruppe im (c)-Schritt beträgt.

3. Verfahren nach Anspruch 2, wobei die Konzentration des Primers der ersten Primergruppe 1/8 bis 1/16 der Konzentration des Primers der zweiten Primergruppe im (c)-Schritt beträgt.

4. Verfahren nach Anspruch 1, wobei der Primer der ersten Primergruppe mindestens ein Primer ist, der aus der Gruppe bestehend aus SEQ ID Nr. 6 und den SEQ ID Nrn. 8-10, ausgewählt ist, und der Primer der zweiten Primergruppe mindestens ein Primer ist, der aus der Gruppe bestehend aus SEQ ID Nr. 5 und SEQ ID Nr. 7 ausgewählt ist.

5. Verfahren zur Genotypisierung eines Targetgens unter Verwendung einer Primerzusammensetzung zur Amplifizierung einer Genregion mit verschiedenen Variationen in einem Targetgen, umfassend:
(a) Amplifizieren des Targetgens nach dem Verfahren nach einem der Ansprüche 1 bis 4;
(b) Exzidieren eines Polynukleotids, das in dem (a)-Schritt amplifiziert wurde, durch Restriktionsenzyme, wobei das Polynukleotid eine Genotyp-analysierbare Sequenz einer genetisch varianten Basissequenz enthält, die der Analyse im Targetgen unterzogen wird;
(c) Erzeugen von mindestens zwei Arten von Einzelstrangpolynukleotidfragmenten mit der Variantenbasis durch Exzidieren der Restriktionsenzyme, wobei die Anzahl an Basen der Fragmente in einem eingeschränkten Bereich liegt; und
(d) Messen der Molekulargewichte der Fragmente.

6. Verfahren nach Anspruch 5, wobei die Anzahl an Basen der Fragmente im Bereich zwischen 2 und 32 liegt.

7. Verfahren nach Anspruch 5, wobei der Primer der ersten Primergruppe mindestens ein Primer ist, der aus der Gruppe bestehend aus SEQ ID Nr. 6 und den SEQ ID Nrn. 8-10 ausgewählt ist, und der Primer der zweiten Primergruppe mindestens ein Primer ist, der aus der Gruppe bestehend aus SEQ ID Nr. 5 und SEQ ID Nr. 7 ausgewählt ist, wenn das in dem (a)-Schritt amplifizierte Gen das HPV-Gen ist.

## Revendications

1. Procédé d'utilisation d'une composition d'amorces pour amplifier une région génique ayant diverses variations et une séquence analysable par génotype dans un gène cible comprenant :
(a) la préparation d'un premier groupe d'amorces comprenant une pluralité d'amorces spécifiques d'une pluralité de matrices, respectivement, chacune des matrices comprend la séquence analysable par génotype et chacune des amorces étant spécifique d'une variation dans le gène cible ;
(b) la préparation d'un deuxième groupe d'amorces comprenant une combinaison d'une amorce sens et d'une amorce antisens, les amorces sens et antisens étant conçues par le choix d'une amorce ayant le plus grand nombre de bases partagées des amorces du premier groupe d'amorces et en utilisant l'amorce choisie comme une référence pour les amorces du premier groupe d'amorces d'avoir la même séquence et longueur que l'amorce choisie, l'amorce sens et/ou l'amorce antisens du deuxième groupe d'amorces se liant spécifiquement à une des matrices et se liant à l'autre des matrices avec un mésappariement permis de jusqu'à trois bases successives ou 1 à 2 bases à proximité de l'extrémité 3' des matrices, dans lequel les amorces du premier groupe d'amorces et du deuxième groupe d'amorces sont complémentaires à une séquence qui flanque la séquence analysable par génotype ;
(c) la réalisation d'une réaction d'amplification par PCR en ajoutant les amorces du premier groupe d'amorces et la combinaison de l'amorce sens et de l'amorce antisens du deuxième groupe d'amorces à un réactif d'amplification comprenant au moins une matrice polynucléotidique, une ADN polymérase, des dNTP et une solution tampon, où le premier groupe d'amorces est ajouté au réactif d'amplification à une faible concentration, et le deuxième groupe d'amorces est ajouté au réactif d'amplification à une concentration élevée par rapport au premier groupe d'amorces ;
(d) la liaison de chaque amorce du premier groupe d'amorces complémentairement à la matrice correspondante, l'amplification de la matrice et l'épuisement du premier groupe d'amorces dans la réaction d'amplification par PCR initiale ;
(e) la réalisation d'une autre réaction d'amplification par la combinaison de l'amorce sens et de l'amorce antisens du deuxième groupe d'amorces, en utilisant les premiers produits d'amplification amplifiés par les amorces du premier groupe d'amorces comme matrices ;
(f) l'obtention des produits d'amplification finaux dont chacun a la séquence analysable par génotype, qui est amplifiée pour avoir la même longueur l'une à l'autre et avoir des séquences nucléotidiques différentes seulement dans la séquence analysable par génotype ; et
dans lequel les produits d'amplification finaux ont la même longueur que les premiers produits d'amplification.

2. Procédé selon la revendication 1, dans lequel la concentration de l'amorce du premier groupe d'amorces est de 1/2 à 1/20 de la concentration de l'amorce du deuxième groupe d'amorces dans l'étape (c).

3. Procédé selon la revendication 2, dans lequel la concentration de l'amorce du premier groupe d'amorces est 1/8 à 1/16 de la concentration de l'amorce du deuxième groupe d'amorces dans l'étape (c).

4. Procédé selon la revendication 1, dans lequel l'amorce du premier groupe d'amorces est au moins une amorce choisie dans le groupe constitué par SEQ ID n° 6 et les SEQ ID n° 8 à 10, et l'amorce du deuxième groupe d'amorces est au moins une amorce choisie dans le groupe constitué par SEQ ID n° 5 et SEQ ID n° 7 quand le gène cible est un gène de HPV.

5. Procédé de génotypage d'un gène cible en utilisant une composition d'amorces pour amplifier une région de gène ayant diverses variations dans un gène cible comprenant :
(a) l'amplification du gène cible selon le procédé de l'une quelconque des revendications 1 à 4 ;
(b) l'excision d'un polynucléotide amplifié dans l'étape (a) par des enzymes de restriction, le polynucléotide comprenant une séquence analysable par génotype d'une séquence de bases variante génétiquement soumise pour être analysée dans le gène cible ;
(c) la production d'au moins deux types de fragments polynucléotidiques simple brin ayant la base variante par l'excision des enzymes de restriction, le nombre de bases des fragment se situant dans une plage limitée ; et
(d) la mesure des poids moléculaires des fragments.

6. Procédé selon la revendication 5, dans lequel le nombre de bases des fragments se situe entre 2 et 32.

7. Procédé selon la revendication 5, dans lequel l'amorce du premier groupe d'amorces est au moins une amorce choisie dans le groupe constitué par SEQ ID n° 6 et les SEQ ID n° 8 à 10, et l'amorce du deuxième groupe d'amorces est au moins une amorce choisie dans le groupe constitué par SEQ ID n° 5 et SEQ ID n° 7 lorsque le gène cible amplifié dans l'étape (a) est un gène de HPV.


<110> GENEMATRIX INC.
   YOO, Wangdon
   HWANG, Sun Young
   SHIN, Soo-Kyung
   SHIN, Ji Young
   LEE, Joo Hyoung
   PARK, Sun Min
   HONG, Sun Pyo
   KIM, Sukjoon
   KIM, Soo-Ok
<120> Primer composition for amplifying genetic region having various genetic variations in target genes, method for amplifying the target genes using the same, PCR amplification kit comprising the same and method for analyzing the genotype of the target genes
<130> P11-0245PCT
<150> KR 10-2010-0043166
   <151> 2010-05-07
<160> 64
<170> KopatentIn 1.71
<210> 1
   <211> 81
   <212> DNA
   <213> Human papillomavirus 59
<400> 1
   gctcagggtt taaacaatgg tatatgttgg cacaatcaat tgtttttaac agttgtagat        60
   actactcgca gcaccaatct t                                                  81
<210> 2
   <211> 81
   <212> DNA
   <213> Human papillomavirus 16
<400> 2
   gcacagggcc acaataatgg catttgttgg ggtaaccaac tatttgttac tgttgttgat        60
   actacacgca gtacaaatat g                                                  81
<210> 3
   <211> 81
   <212> DNA
   <213> Human papillomavirus 51
<400> 3
   gcgcagggtc acaataatgg catttgctgg aacaatcagc tttttattac ctgtgttgat        60
   actaccagaa gtacaaattt a                                                  81
<210> 4
   <211> 81
   <212> DNA
   <213> Human papillomavirus 68
<400> 4
   gcacagggac acaacaatgg tatttgttgg cataatcaat tatttcttac tgttgtggat        60
   accactcgca gtaccaattt t 81
<210> 5
   <211> 24
   <212> DNA
   <213> Artificial Sequence
<220>
   <223> HPV primer 1
<400> 5
   gcacagggcc acaaggatga atgg 24
<210> 6
   <211> 24
   <212> DNA
   <213> Artificial Sequence
<220>
   <223> HPV primer 2
<400> 6
   gcacagggtt taaaggatga atgg 24
<210> 7
   <211> 26
   <212> DNA
   <213> Artificial Sequence
<220>
   <223> HPV primer 3
<400> 7
   gtagtatcaa caacggatgt aacaaa 26
<210> 8
   <211> 26
   <212> DNA
   <213> Artificial Sequence
<220>
   <223> HPV primer 4
<400> 8
   gtagtatcaa caacggatgt taaaaa 26
<210> 9
   <211> 26
   <212> DNA
   <213> Artificial Sequence
<220>
   <223> HPV primer 5
<400> 9
   gtagtatcaa caacggatgt aataaa 26
<210> 10
   <211> 26
   <212> DNA
   <213> Artificial Sequence
<220>
   <223> HPV primer 6
<400> 10
   gtagtatcaa caacggatgt aagaaa 26
<210> 11
   <211> 73
   <212> DNA
   <213> Artificial Sequence
<220>
   <223> HPV 59 amplified DNA fragment
<400> 11
   gcacagggtt taaaggatga atggtatatg ttggcacaat caattgtttt ttaacatccg 60
   ttgttgatac tac 73
<210> 12
   <211> 72
   <212> DNA
   <213> Artificial Sequence
<220>
   <223> HPV 16 amplified DNA fragment
<400> 12
   gcacagggcc acaaggatga atggcatttg ttggggtaac caactatttg ttacatccgt 60
   tgttgatact ac 72
<210> 13
   <211> 72
   <212> DNA
   <213> Artificial Sequence
<220>
   <223> HPV 51 amplified DNA fragment
<400> 13
   gcacagggcc acaaggatga atggcatttg ctggaacaat cagcttttta ttacatccgt 60
   tgttgatact ac 72
<210> 14
   <211> 72
   <212> DNA
   <213> Artificial Sequence
<220>
   <223> HPV 68 amplified DNA fragment
<400> 14
   gcacagggcc acaaggatga atggtatttg ttggcataat caattatttc ttacatccgt 60
   tgttgatact ac 72
<210> 15
   <211> 73
   <212> DNA
   <213> Artificial Sequence
<220>
   <223> HPV 59 amplified DNA fragment
<400> 15
   gcacagggcc acaaggatga atggtatatg ttggcacaat caattgtttt gttacatccg 60
   ttgttgatac tac 73
<210> 16
   <211> 72
   <212> DNA
   <213> Artificial Sequence
<220>
   <223> HPV 16 amplified DNA fragment
<400> 16
   gcacagggcc acaaggatga atggcatttg ttggggtaac caactatttg ttacatccgt 60
   tgttgatact ac 72
<210> 17
   <211> 72
   <212> DNA
   <213> Artificial Sequence
<220>
   <223> HPV 51 amplified DNA fragment
<400> 17
   gcacagggcc acaaggatga atggcatttg ctggaacaat cagctttttg ttacatccgt 60
   tgttgatact ac 72
<210> 18
   <211> 72
   <212> DNA
   <213> Artificial Sequence
<220>
   <223> HPV 68 amplified DNA fragment
<400> 18
   gcacagggcc acaaggatga atggtatttg ttggcataat caattatttg ttacatccgt          60
   tgttgatact ac                                                              72
<210> 19
   <211> 83
   <212> DNA
   <213> Human HLA-DQB1 *0501
<400> 19
   tgtgctactt caccaacggg acggagcgcg tgcggggtgt gaccagacac atctataacc          60
   gagaggagta cgtgcgcttc gac                                                  83
<210> 20
   <211> 83
   <212> DNA
   <213> Human HLA-DQB1 *0201
<400> 20
   tgtgctactt caccaacggg acagagcgcg tgcgtcttgt gagcagaagc atctataacc          60
   gagaagagat cgtgcgcttc gac                                                  83
<210> 21
   <211> 83
   <212> DNA
   <213> Artificial Sequence
<220>
   <223> Human HLA-DQB1 *0401
<400> 21
   tgtgctactt caccaacggg accgagctcg tgcggggtgt gaccagatac atctataacc          60
   gagaggagta cgcgcgcttc gac                                                  83
<210> 22
   <211> 83
   <212> DNA
   <213> Human HLA-DQB1 *0301
<400> 22
   tgtgctactt caccaacggg acggagcgcg tgcgttatgt gaccagatac atctataacc 60
   gagaggagta cgcacgcttc gac 83
<210> 23
   <211> 30
   <212> DNA
   <213> Artificial Sequence
<220>
   <223> HLA-DQB1 primer 7
<400> 23
   tgtgctactt caccaacgga tggacggagc 30
<210> 24
   <211> 33
   <212> DNA
   <213> Artificial Sequence
<220>
   <223> HLA-DQB1 primer 8
<400> 24
   gcgtgcgtac tcctctcggt ggatgataga tgt 33
<210> 25
   <211> 30
   <212> DNA
   <213> Artificial Sequence
<220>
   <223> HLA-DQB1 primer 9
<400> 25
   tgtgctactt caccaacgga tggacagagc 30
<210> 26
   <211> 33
   <212> DNA
   <213> Artificial Sequence
<220>
   <223> HLA-DQB1 primer 10
<400> 26
   gcgcacgatc tcttctcggt ggatgataga tgc 33
<210> 27
   <211> 84
   <212> DNA
   <213> Artificial Sequence
<220>
   <223> HLA-DQB1 *0201 amplified DNA fragment
<400> 27
   tgtgctactt caccaacgga tggacagagc gcgtgcgtct tgtgagcaga agcatctatc 60
   atccaccgag aagagatcgt gcgc 84
<210> 28
   <211> 84
   <212> DNA
   <213> Artificial Sequence
<220>
   <223> HLA-DQB1 *0501 amplified DNA fragment
<400> 28
   tgtgctactt caccaacgga tggacggagc gcgtgcgggg tgtgaccaga cacatctatc 60
   atccaccgag aggagtacgc acgc 84
<210> 29
   <211> 84
   <212> DNA
   <213> Artificial Sequence
<220>
   <223> HLA-DQB1 *0201 amplified DNA fragment
<400> 29
   tgtgctactt caccaacgga tggacggagc gcgtgcgtct tgtgagcaga aacatctatc 60
   atccaccgag aagagatcgc acgc 84
<210> 30
   <211> 84
   <212> DNA
   <213> Artificial Sequence
<220>
   <223> HLA-DQB1 *0401 amplified DNA fragment
<400> 30
   tgtgctactt caccaacgga tggacggagc tcgtgcgggg tgtgaccaga tacatctatc 60
   atccaccgag aggagtacgc acgc 84
<210> 31
   <211> 84
   <212> DNA
   <213> Artificial Sequence
<220>
   <223> HLA-DQB1 *0301 amplified DNA fragment
<400> 31
   tgtgctactt caccaacgga tggacggagc gcgtgcgtta tgtgaccaga tacatctatc 60
   atccaccgag aggagtacgc acgc 84
<210> 32
   <211> 60
   <212> DNA
   <213> HBV # 1
<400> 32
   taccaatttt ctttttgtct ttgggtatac atttaaaccc taataaaacc aaacgttggg 60 60
<210> 33
   <211> 60
   <212> DNA
   <213> HBV # 2
<400> 33
   taccaatttt ctttttgtct ttgggtatac atttaaaccc tactaaaacc aaacgttggg 60 60
<210> 34
   <211> 60
   <212> DNA
   <213> HBV # 3
<400> 34
   taccaatttt ctttttgtct ttgggtatac atttaaaccc tgataaaacc aaacgttggg 60 60
<210> 35
   <211> 60
   <212> DNA
   <213> HBV # 4
<400> 35
   taccaatttt ctttttgtct ttgggtatac atttaaaccc tcataaaacc aaacgttggg 60 60
<210> 36
   <211> 60
   <212> DNA
   <213> HBV # 5
<400> 36
   taccaatttt ctttttgtct ttgggtatac atttaaacca taataaaacc aaacgttggg 60 60
<210> 37
   <211> 60
   <212> DNA
   <213> HBV # 6
<400> 37
   taccaatttt ctttttgtct ttgggtatac atttaaacca tactaaaacc aaacgttggg 60 60
<210> 38
   <211> 60
   <212> DNA
   <213> HBV # 7
<400> 38
   taccaatttt ctttttgtct ttgggtatac atttaaacca tgataaaacc aaacgttggg 60 60
<210> 39
   <211> 60
   <212> DNA
   <213> HBV # 8
<400> 39
   taccaatttt ctttttgtct ttgggtatac atttaaacca tcataaaacc aaacgttggg 60 60
<210> 40
   <211> 39
   <212> DNA
   <213> Artificial Sequence
<220>
   <223> HBV primer 11
<400> 40
   taccaatttt cttttggatg tgtctttggg tatacattt 39
<210> 41
   <211> 27
   <212> DNA
   <213> Artificial Sequence
<220>
   <223> HBV primer 12
<400> 41
   cccaacgttt ggatgggttt tattagg 27
<210> 42
   <211> 27
   <212> DNA
   <213> Artificial Sequence
<220>
   <223> HBV primer 13
<400> 42
   cccaacgttt ggatgggttt tagtagg 27
<210> 43
   <211> 27
   <212> DNA
   <213> Artificial Sequence
<220>
   <223> HBV primer 14
<400> 43
   cccaacgttt ggatgggttt tatcagg 27
<210> 44
   <211> 27
   <212> DNA
   <213> Artificial Sequence
<220>
   <223> HBV primer 15
<400> 44
   cccaacgttt ggatgggttt tatgagg 27
<210> 45
   <211> 27
   <212> DNA
   <213> Artificial Sequence
<220>
   <223> HBV primer 16
<400> 45
   cccaacgttt ggatgggttt tattatg 27
<210> 46
   <211> 27
   <212> DNA
   <213> Artificial Sequence
<220>
   <223> HBV primer 17
<400> 46
   cccaacgttt ggatgggttt tagtatg 27
<210> 47
   <211> 27
   <212> DNA
   <213> Artificial Sequence
<220>
   <223> HBV primer 18
<400> 47
   cccaacgttt ggatgggttt tatcatg 27
<210> 48
   <211> 27
   <212> DNA
   <213> Artificial Sequence
<220>
   <223> HBV primer 19
<400> 48
   cccaacgttt ggatgggttt tatgatg 27
<210> 49
   <211> 70
   <212> DNA
   <213> Artificial Sequence
<220>
   <223> HBV # 1 amplified DNA fragment
<400> 49
   taccaatttt cttttggatg tgtctttggg tatacattta aaccctaata aaacccatcc 60
   aaacgttggg 70
<210> 50
   <211> 70
   <212> DNA
   <213> Artificial Sequence
<220>
   <223> HBV # 2 amplified DNA fragment
<400> 50
   taccaatttt cttttggatg tgtctttggg tatacattta aaccctacta aaacccatcc 60
   aaacgttggg 70
<210> 51
   <211> 70
   <212> DNA
   <213> Artificial Sequence
<220>
   <223> HBV # 3 amplified DNA fragment
<400> 51
   taccaatttt cttttggatg tgtctttggg tatacattta aaccctgata aaacccatcc 60
   aaacgttggg 70
<210> 52
   <211> 70
   <212> DNA
   <213> Artificial Sequence
<220>
   <223> HBV # 4 amplified DNA fragment
<400> 52
   taccaatttt cttttggatg tgtctttggg tatacattta aaccctcata aaacccatcc 60
   aaacgttggg 70
<210> 53
   <211> 70
   <212> DNA
   <213> Artificial Sequence
<220>
   <223> HBV # 5 amplified DNA fragment
<400> 53
   taccaatttt cttttggatg tgtctttggg tatacattta aaccataata aaacccatcc 60
   aaacgttggg 70
<210> 54
   <211> 70
   <212> DNA
   <213> Artificial Sequence
<220>
   <223> HBV # 6 amplified DNA fragment
<400> 54
   taccaatttt cttttggatg tgtctttggg tatacattta aaccatacta aaacccatcc 60
   aaacgttggg 70
<210> 55
   <211> 70
   <212> DNA
   <213> Artificial Sequence
<220>
   <223> HBV # 7 amplified DNA fragment
<400> 55
   taccaatttt cttttggatg tgtctttggg tatacattta aaccatgata aaacccatcc 60
   aaacgttggg 70
<210> 56
   <211> 70
   <212> DNA
   <213> Artificial Sequence
<220>
   <223> HBV # 8 amplified DNA fragment
<400> 56
   taccaatttt cttttggatg tgtctttggg tatacattta aaccatcata aaacccatcc 60
   aaacgttggg 70
<210> 57
   <211> 70
   <212> DNA
   <213> Artificial Sequence
<220>
   <223> HBV # 1 amplified DNA fragment
<400> 57
   taccaatttt cttttggatg tgtctttggg tatacattta aaccctaata aaacccatcc 60
   aaacgttggg 70
<210> 58
   <211> 70
   <212> DNA
   <213> Artificial Sequence
<220>
   <223> HBV # 2 amplified DNA fragment
<400> 58
   taccaatttt cttttggatg tgtctttggg tatacattta aaccctaata aaacccatcc 60
   aaacgttggg 70
<210> 59
   <211> 70
   <212> DNA
   <213> Artificial Sequence
<220>
   <223> HBV # 3 amplified DNA fragment
<400> 59
   taccaatttt cttttggatg tgtctttggg tatacattta aaccctaata aaacccatcc 60
   aaacgttggg 70
<210> 60
   <211> 70
   <212> DNA
   <213> Artificial Sequence
<220>
   <223> HBV # 4 amplified DNA fragment
<400> 60
   taccaatttt cttttggatg tgtctttggg tatacattta aaccctaata aaacccatcc 60
   aaacgttggg 70
<210> 61
   <211> 70
   <212> DNA
   <213> Artificial Sequence
<220>
   <223> HBV # 5 amplified DNA fragment
<400> 61
   taccaatttt cttttggatg tgtctttggg tatacattta aaccctaata aaacccatcc 60
   aaacgttggg 70
<210> 62
   <211> 70
   <212> DNA
   <213> Artificial Sequence
<220>
   <223> HBV # 6 amplified DNA fragment
<400> 62
   taccaatttt cttttggatg tgtctttggg tatacattta aaccctaata aaacccatcc 60
   aaacgttggg 70
<210> 63
   <211> 70
   <212> DNA
   <213> Artificial Sequence
<220>
   <223> HBV # 7 amplified DNA fragment
<400> 63
   taccaatttt cttttggatg tgtctttggg tatacattta aaccctaata aaacccatcc 60
   aaacgttggg 70
<210> 64
   <211> 70
   <212> DNA
   <213> Artificial Sequence
<220>
   <223> HBV # 8 amplified DNA fragment
<400> 64
   taccaatttt cttttggatg tgtctttggg tatacattta aaccctaata aaacccatcc       60
   aaacgttggg                                                              70